(19) Europäisches Patentamt
European Patent Office
Office européen des brevets

(11) Publication number: **0 524 813 A1**

(12) **EUROPEAN PATENT APPLICATION**

(21) Application number: **92306711.0**

(51) Int. Cl.$^5$: **C07J 31/00**

(22) Date of filing: **22.07.92**

(30) Priority: **25.07.91 US 735509**
**25.07.91 US 735962**
**25.07.91 US 735981**

(43) Date of publication of application:
**27.01.93 Bulletin 93/04**

(84) Designated Contracting States:
**AT BE CH DE DK ES FR GB GR IT LI LU MC NL PT SE**

(71) Applicant: **E.R. SQUIBB & SONS, INC.**
**Lawrenceville-Princeton Road**
**Princeton New Jersey 08543-4000 (US)**

(72) Inventor: **Delaney, Edward J.**
**137 Cherrybrook Drive**
**Princeton, New Jersey (US)**
Inventor: **Domina, Daniel J.**
**6 Meadow Lane**
**East Windsor, New Jersey (US)**
Inventor: **Taylor, Stephan P.B.**
**1 Cambridge Way**
**East Windsor, New Jersey (US)**
Inventor: **Korzun, John N.**
**1201 Williamson Road**
**North Brunswick, New Jersey (US)**

(74) Representative: **Thomas, Roger Tamlyn et al**
**D. Young & Co. 10 Staple Inn**
**London WC1V 7RD (GB)**

(54) **Selective thioketalization process, process for preparing thioenol ethers from thioketals, and process for the preparation of thioketals.**

(57) A selective thioketalization process for the preparation of a thioketal from a dione where one, but not the other, keto group of the dione is α,β-unsaturated, and where selective thioketalization occurs at the keto group of the dione which is not α,β-unsaturated, comprising the step of contacting the dione with a thiol, in the presence of a protic acid catalyst which is a complex of a Lewis acid and a protic source, wherein the temperature of the reaction medium is maintained below about 25°C during the reaction. Preferably, further, (i) the acid catalyst is employed in excess and/or (ii) the thiol is employed in excess. The inventive process may be employed, for example, in the preparation of a 3-keto,17-thioketalandrostene from the corresponding 3,17-diketoandrostene.

A process for the preparation of thioenolethers from thioketals, especially 3-keto,17-thioenoletherandrostenes from 3-keto,17-thioketalandrostenes, wherein the thioketal is contacted with a protic acid catalyst.

A process for the preparation of thioketals, especially mixed thioketals such as mixed 3-keto,17-thioketalandrostenes, from thioenolethers, comprising the step of contacting a thioenolether with a thiol in the presence of a protic acid catalyst.

EP 0 524 813 A1

The instant invention relates to a new process for the selective thioketalization of diones where one, but not the other, keto group is α,β-unsaturated, especially to the preparation of 3-keto,17-thioketalandrostenes from 3,17-diketoandrostenes. The compounds produced by the instant process, and derivatives thereof, may be used, for example, as antiinflammatory agents.

The instant invention also relates to a new process for the preparation of thioenolethers from thioketals, especially to the preparation of 3-keto,17-thioenoletherandrostenes from 3-keto,17-thioketalandrostenes. The compounds produced by the instant process may be used, for example, as intermediates in the preparation of antiinflammatory agents.

The instant invention further relates to a new process for the preparation of thioketals from thioenolethers, especially to the stereoselective preparation of mixed thioketals such as mixed 3-keto,17-thioketalandrostenes from 3-keto,17-thioenoletherandrostenes. The compounds produced by the instant process, or derivatives thereof, may be used, for example, as antiinflammatory agents.

Processes for the preparation of thioketals, such as 3-keto,17-thioketalandrostenes, which are selective with respect to the products formed are desirable. Also desirable are processes for the preparation of thioenolethers such as 3-keto,17-thioenoletherandrostenes which may be employed efficiently, with sufficient reaction rate, at lower temperatures, avoiding the formation of undesirable quantities of degradation products, as well as at higher temperatures. Additionally, processes for the preparation of thioketals which run efficiently, and which further provide good stereoselectivity at higher, as well as at lower temperatures, are desirable.

The instant invention provides a selective thioketalization process for the preparation of a thioketal from a dione where one, but not the other, keto group of the dione is α,β-unsaturated, and where selective thioketalization occurs at the keto group of the dione which is not α,β-unsaturated, comprising the step of contacting the dione with a thiol, in the presence of a protic acid catalyst which is a complex of a Lewis acid and a protic source, wherein the temperature of the reaction medium is maintained below about 25°C during the reaction. In conducting the process of the instant invention it is further preferred that (i) the acid catalyst is employed in excess, and/or (ii) the thiol is employed in excess. The instant invention provides, for example, a process for the preparation of a 3-keto,17-thioketalandrostene (e.g., where both thiol groups at the 17-position are the same) from a 3,17-diketoandrostene, where a highly regioselective reaction at the 17-position is achieved. Preferred embodiments of the instant selective thioketalization process are described below.

The instant invention also provides a process for the preparation of a thioenolether, such as a 3-keto,17-thioenoletherandrostene, from a thioketal, such as a 3-keto,17-thioketalandrostene (e.g., where both thiol groups at the 17-position are the same), comprising the step of contacting the thioketal with a protic acid catalyst. By use of the instant process, efficient preparation of the thioenolether may be achieved even at relatively low temperatures. Preferred embodiments of the instant process for preparing thioenol ethers from thioketals are described below.

The instant invention further provides a process for the preparation of a thioketal from a thioenolether, comprising the step of contacting the thioenolether with a thiol in the presence of a protic acid catalyst. A preferred embodiment of the instant invention provides a process for the stereoselective preparation of a mixed thioketal, such as a mixed 3-keto,17-thioketalandrostene. Preferred embodiments of the instant process for the preparation of thioketals are described below.

## Definitions

A broken line in the 1,2-, 6,7- or 15,16-position of a structural formula in this specification indicates the optional presence of ethylenic unsaturation.

The term "aryl", as used throughout this specification either individually or as part of a larger group, preferably refers to phenyl or phenyl substituted with one or two alkyl, alkoxy or halogen groups.

The term "halogen", as used throughout this specification either individually or as part of a larger group, refers to fluorine, chlorine, bromine and iodine.

The term "alkyl", as used throughout this specification either individually or as part of a larger group, preferably refers to groups having 1 to 12 carbon atoms.

The term "cycloalkyl", as used throughout this specification, either individually or as part of a larger group, preferably refers to groups having 3, 4, 5, 6 or 7 carbon atoms.

The term "androstene," as used throughout this specification, refers to androstanes having ethylenic unsaturation in one or more positions. Exemplary of androstenes specifically contemplated are $\Delta^{4}$-androstenes, $\Delta^{1,4}$-androstadienes, $\Delta^{4,6}$-androstadienes, and $\Delta^{1,4,6}$-androstatrienes, and, where appropriate, $\Delta^{1,4,15}$-androstatrienes, $\Delta^{4,6,15}$-androstatrienes and $\Delta^{1,4,6,15}$-androstatetraenes.

The term "mixed", as used in this specification in reference to the compounds produced by the inventive process, denotes thiol groups of the thioketal which differ from each other.

The term "thioketal", as used throughout this specification, refers to the moiety, or to compounds containing the moiety,

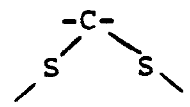

.

The term "thioenol ether", as used throughout this specification, refers to the moiety, or the compounds containing the moiety,

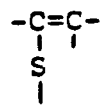

.

The term "dione", as used throughout this specification, refers to compounds having two or more keto groups where one keto group is α,β-unsaturated and one keto group is not.

An "excess" quantity of protic acid catalyst, as used throughout this specification, refers to a molar ratio of acid catalyst:dione (such as 3,17-diketoandrostene) starting material greater than about 1:1. Preferred as excess quantities are those molar ratios from about 2:1 to about 10:1, most preferably about 5:1. The molar ratio of acid catalyst:starting material may, for example, be calculated as the molar ratio of Lewis acid:starting material when the Lewis acid employed is the limiting component in forming a Lewis acid/protic source complex.

An "excess" quantity of thiol, as used throughout this specification, refers to a molar ratio of thiol:dione (such as 3,17-diketoandrostene) starting material greater than about 2:1. Preferably, a molar ratio of from about 3:1 to about 10:1 is employed, most preferably a ratio of about 6:1.

"Selective thioketalization", as used throughout this specification, refers to preferential formation of a thioketal moiety on the keto group which is not α,β-unsaturated on the dione starting material. Preferably, the formation of a thioketal moiety on the α,β-unsaturated keto group is substantially absent.

The definition given for a group or moiety applies throughout this specification, unless otherwise indicated.

Throughout the specification, the rings are lettered and the positions are numbered as follows:

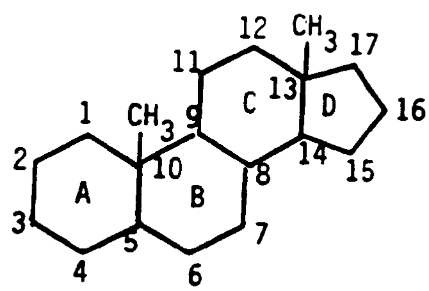

The 11-hydroxyl groups of the compounds of the formulae I, II, IV, V and VI following are optionally protected. Groups employed for the protection of these hydroxyl groups may be any of those groups known in the art as hydroxyl protecting groups. An exemplary family of protecting groups is the acyl family, especially alkanoyl groups such as acetyl. Means for protection and deprotection of hydroxyl groups are well known in the art. To convert the 11-hydroxyl group to an 11-acetyloxy group, for example, it is preferred to react the former group with acetic acid anhydride in the presence of 4-dimethylaminopyridine (4-DMAP). Protection of the 11-hydroxyl groups may provide compounds with enhanced solubility, facilitating the reaction and/or subsequent isolation and purification procedures.

## Selective Thioketalization Process

In a preferred embodiment, the instant invention provides a process for the preparation of a 3-keto,17-thioketalandrostene from the corresponding 3,17-diketoandrostene, comprising the step of contacting the 3,17-diketoandrostene with a thiol, in the presence of a protic acid catalyst which is a complex of a Lewis acid and

a protic source, wherein the temperature of the reaction medium is maintained below about 25°C during the reaction. In a particularly preferred embodiment, the instant invention provides a process for the preparation of a 3-keto,17-thioketalandrostene of the formula I:

(I)

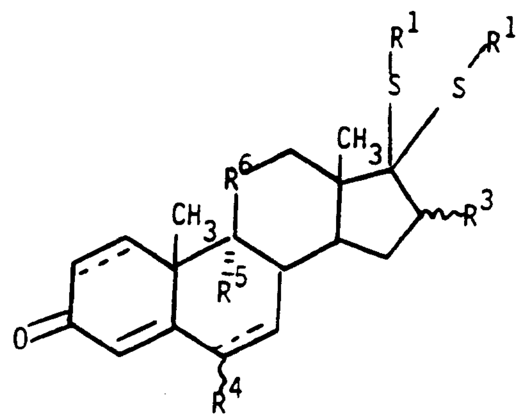

where

$R^1$ is alkyl, cycloalkyl or aryl, and both $R^1$ groups are the same;

$R^3$ is hydrogen, hydroxy, alkoxy, aryloxy, alkylthio, arylthio, alkylcarbonyloxy or halogen;

$R^4$ is hydrogen, alkyl, hydroxy, alkylcarbonyloxy or halogen;

$R^5$ is hydrogen or halogen; and

$R^6$ is carbonyl, (β-hydroxy)methine, or protected (β-hydroxy)methine,

comprising the step of contacting a 3,17-diketoandrostene of the formula II:

(II)

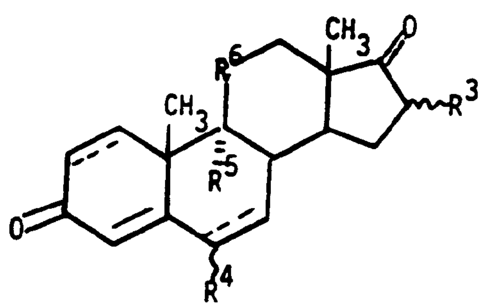

where $R^3$, $R^4$, $R^5$ and $R^6$ are as defined above for formula I, with a thiol of the formula III:

$R^1$-SH (III)

where $R^1$ is as defined above for formula I, in the presence of a protic acid catalyst which is a complex of a Lewis acid and a protic source, wherein the temperature of the reaction medium is maintained below about 25°C during the reaction.

Those compounds of the formula I where $R^6$ is carbonyl or, especially, (β-hydroxy)methine are particularly useful as antiinflammotory agents, and are preferred. Such compounds may be formed directly or by deprotection of a compound of the formula I prepared where $R^6$ is protected (β-hydroxy)methine. It is particularly preferred to prepare, according to the process of the instant invention, those compounds of the formula I which are $\Delta^{1,4}$-androstadienes in which

$R^1$ is alkyl, especially methyl;

$R^3$ and $R^4$ are hydrogen;

$R^5$ is halogen, especially fluoro; and

$R^6$ is (β-hydroxy)methine.

Thus, an especially preferred embodiment of the instant process relates to the preparation of a 3-keto,17-thioketalandrostene of the formula Ia:

(Ia)

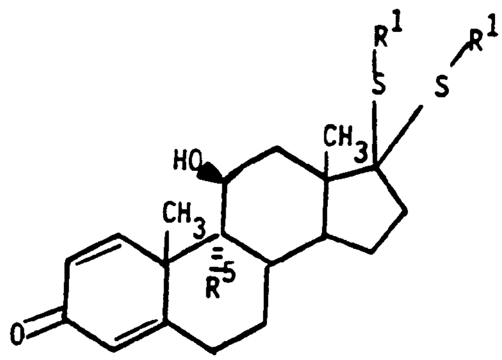

where

$R^1$ is alkyl, and both $R^1$ groups are the same; and

$R^5$ is halogen, especially fluoro;

comprising the step of contacting a 3,17-diketoandrostene of the formula IIa:

(IIa)

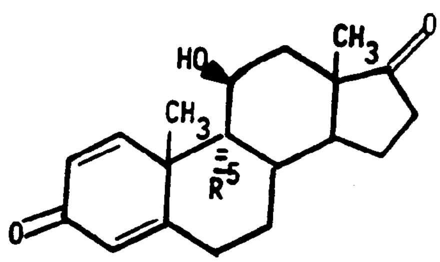

where $R^5$ is as defined above for formula Ia, with a thiol of the formula IIIa:

$R^1$-SH (IIIa)

where $R^1$ is as defined above for formula Ia, in the presence of a protic acid catalyst which is a complex of a Lewis acid and a protic source, wherein the temperature of the reaction medium is maintained below about 25°C during the reaction.

Preferably, conditions (i) (excess acid) or (ii) (excess thiol), most preferably both conditions (i) and (ii), are employed in all embodiments of the instant process.

The starting 3,17-diketoandrostenes of the formula II may be obtained by methods known in the art, such as by the methods set forth in U.S. Patent No. 4,361,559, referred to above, and incorporated herein by reference.

Thiols of the formula III may be obtained by methods known to those skilled in the art. The thiol employed may function as a solvent, facilitating dissolution of the 3,17-diketoandrostene starting material. The thiol may also function as a protic source for certain Lewis acids such as boron trifluoride. The use of an additional protic source is, however, preferred.

Any Lewis acid/protic source complex enhancing the reaction rate, as compared with the process conducted under the same conditions in the absence of the complex, may be employed as the catalyst of the instant process. The catalyst may be employed alone or in conjunction with one or more additional Lewis acid/protic source catalysts.

Exemplary Lewis acids used in the complexes of the instant invention include boron trifluoride or, especially, etherates thereof such as boron trifluoride ethyl etherate ($BF_3Et_2O$), as well as Lewis acids such as $SnCl_4$, $TiCl_4$, $BBr_3$, $BCl_3$ and the like. Exemplary protic sources used in such complexes include oxygen nucleophiles

bearing an acidic proton such as water, organic alcohols, particularly alkanols such as methyl or ethyl alcohol, and organic protic acids such as organic carboxylic acids, especially alkyl carboxylic acids such as acetic acid and trifluoroacetic acid, alone or in combination with the anhydride thereof. It is preferred that water, such as trace water, not be the sole protic source employed. An alkyl carboxylic acid such as acetic acid complexed with boron trifluoride or an etherate thereof such as boron trifluoride ethyl etherate is preferred as the Lewis acid/protic source complex.

A pre-formed Lewis acid/protic source complex may be added directly. Preferably, however, at least part of the Lewis acid and/or protic source is added separately, so that the complex is formed in situ.

It is preferred to employ input materials which are substantially, preferably completely, free of water to minimize or eliminate hydrolysis. In this regard, a precursor of the protic acid catalyst may be employed which, in combination with water, provides the acid catalyst of the inventive process while removing water as an effective hydrolyzing agent. Exemplary precursors include the Lewis acid of the Lewis acid/protic source complexes. Addition of a protic source other than water, preferably one with less affinity for the Lewis acid than water, is desirable to ensure a sufficient quantity of acid catalyst to the extent that moisture is absent from the input components.

The preferred order of addition of the reaction components of the instant process is that where the acid catalyst, or Lewis acid component, is added to a previously prepared mixture of 3,17-diketoandrostene starting material, thiol and, optionally, solvent.

It is preferred in the instant process to maintain the temperature of the reaction medium below about 20°C, especially below about 15°C, during the reaction. It is particularly preferred to maintain the temperature of the reaction medium below about 10°C, such as where the maximum temperature is no greater than about 6 to 8°C, during the reaction. It is also preferred, in addition to maintaining the temperature during the reaction below a given level, to minimize the increase in the temperature during the reaction.

Temperature control is preferably achieved by pre-cooling one or more of the components of the reaction medium. Chilling the acid catalyst, including chilling the Lewis acid of the Lewis acid/protic source complex, is preferred prior to addition thereof to the other reaction components. The chilled catalyst acts as an effective heat sink, facilitating temperature control.

Temperature control may also be achieved by adding the catalyst, preferably chilled as above, to a chilled mixture of 3,17-diketoandrostene starting material, thiol and, optionally, solvent. It is particularly preferred to chill the catalyst to a temperature below that of the mixture of starting material, thiol and, optionally, solvent. Preferably, the latter mixture is cooled to a temperature between about 15°C and the freezing point of the mixture, although partial freezing of the mixture may be employed. Partial freezing is less preferred as the heat generated by the reaction may be absorbed to result in only a partial thaw of the mixture. Thus, any portion of the starting material engulfed in solids may remain unreacted, with longer reaction times needed to allow thawing.

The starting material is preferably completely dissolved prior to commencing the reaction.

Reaction conditions such as the pressure and nature of the atmosphere employed may be selected by the skilled artisan.

The reaction may optionally be run in an organic solvent, for example, a halogenated hydrocarbon such as methylene chloride, or a mixture of organic solvents. Preferably, a solvent is employed in which the product is insoluble, thus forcing precipitation of product during the reaction. Precipitation of the product formed minimizes the opportunity for overreaction thereof. In this regard, the reaction solvent may be seeded with product to further enhance product precipitation.

The amount and nature of the solvent is also preferably chosen to minimize the freezing point of the reaction medium, so that the reaction temperature employed may also be minimized without freezing of the reaction mixture components. Most preferably, the reaction medium should be as concentrated as possible, as excess solvent slows the reaction, while still employing the solvent in a sufficient quantity to permit complete dissolution of the 3,17-diketoandrostene starting material prior to commencing the reaction.

A solvent is preferably employed which serves both as a solvent and as the protic source of the Lewis acid/protic source complex. Exemplary such solvents are alkyl carboxylic acids such as acetic acid.

The process of the instant invention is advantageous in achieving a product of high purity in high yield. Temperature control according to the instant invention, preferably with the use of excess acid catalyst and/or excess thiol, allows selective thioketalization of a dione at the keto group which is not α,β-unsaturated, minimizing or eliminating thioketalization at the α,β-unsaturated keto group.

For example, the 3- and 17-positions, inter alia, of a 3,17-diketoandrostene may react when in contact with a thiol and a protic acid catalyst. In the absence of the conditions of the process of the instant invention, undesirable quantities of side-products such as:

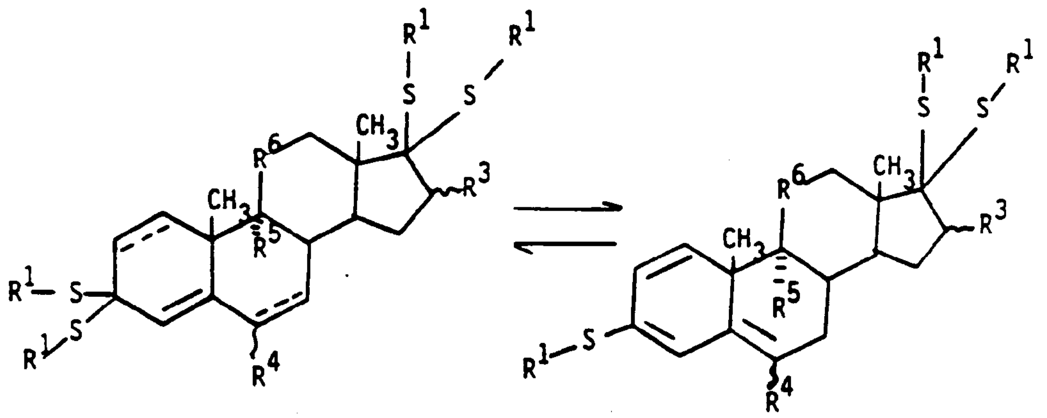

and/or

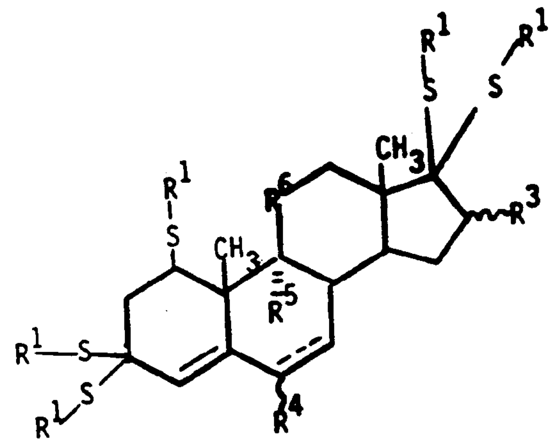

in which the substituents are defined as above for formula I, as well as other colored decomposition products, may be formed. Formation of such unwanted side-products reduces the yield of the desired product, and complicates the subsequent isolation and purification steps.

Thioketalization according to the instant process, however, allows achievement of a highly regioselective reaction at the 17-position. Thus, the instant process allows preparation of a 3-keto,17-thioketalandrostene of the formula I in which the formation of unwanted side-products is minimized or eliminated, improving the yield and purity of the product obtained, and allowing simplified product isolation and purification steps to be employed. Reaction conditions are preferably selected when conducting the instant process so as to prepare a compound of the formula I substantially free of such unwanted side-products.

The reaction of the instant process is generally rapid, occurring within a span of several minutes. The solvent and reaction conditions are preferably selected so that the product crystallizes from solution during that time. The reaction is preferably halted when the starting material is substantially consumed, as allowing additional reaction time may result in overaddition of any product remaining in solution. Thus, the yield and purity of the product obtained may be further enhanced by minimizing the reaction time. The reaction time is preferably minimized by rapid addition of the catalyst to the reactants, such as by addition of the catalyst, or the Lewis acid component of the Lewis acid/protic source complex, over the course of a time period less than one minute, most preferably less than 20, and especially less than 10, seconds.

Upon substantial completion thereof, the reaction may be quenched, preferably by addition of an amount of a liquid such as water which concomitantly crystallizes any product remaining dissolved. The product may then be reslurried one or more times as desired to remove any impurities which may be present. Isopropanol near its reflux temperature is preferably employed for reslurrying. The volume of liquid such as isopropanol should be selected to minimize the loss of product.

The preferred methods for preparing 3-keto,17-thioketalandrostenes described herein may be employed as appropriate for the preparation of any thioketal according to the instant process.

Preparing Thioenol Ethers from Thioketals

The instant invention provides a process for the preparation of a thioenolether from a thioketal, comprising the step of contacting the thioketal with a protic acid catalyst. Efficient preparation of a thioenolether may preferably be achieved where the protic acid catalyst is formed, at least in part, by addition of a protic acid catalyst precursor to a protic source, where the reaction rate is controlled and/or where the thiol released is removed during the reaction.

A particularly preferred embodiment of the instant invention provides a process for the preparation of a 3-keto,17-thioenoletherandrostene of the formula IV:

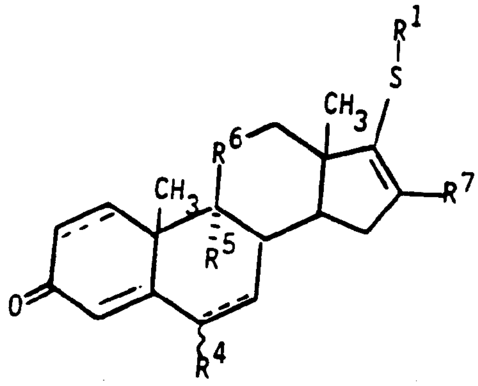

(IV)

where

$R^1$ is alkyl, cycloalkyl or aryl;

$R^7$ is hydrogen, alkoxy, aryloxy, alkylthio, arylthio, alkylcarbonyloxy or halogen;

$R^4$ is hydrogen, alkyl, hydroxy, alkylcarbonyloxy or halogen;

$R^5$ is hydrogen or halogen; and

$R^6$ is carbonyl, (β-hydroxy)methine, or protected (β-hydroxy)methine,

comprising the step of contacting a 3-keto,17-thioketalandrostene of the formula V:

(V)

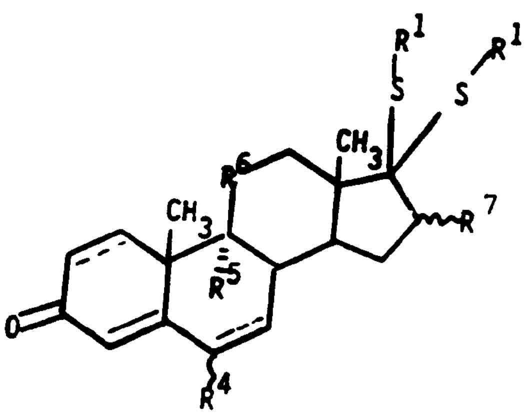

where $R^1$, $R^7$, $R^4$, $R^5$ and $R^6$ are as defined above for formula IV and both $R^1$ groups are the same, with a protic acid catalyst.

It is particularly preferred to prepare, according to the process of the instant invention, those compounds of the formula IV which are $\Delta^{1,4}$-androstadienes in which:

$R^1$ is alkyl, especially methyl;

$R^7$ and $R^4$ are hydrogen;

$R^5$ is halogen, especially fluoro; and

$R^6$ is protected (β-hydroxy)methine, especially alkylcarbonyloxymethine such as acetyloxymethine.

Thus, an especially preferred embodiment of the instant invention relates to a process for the preparation of a 3-keto,17-thioenoletherandrostene of the formula IVa:

(IVa)

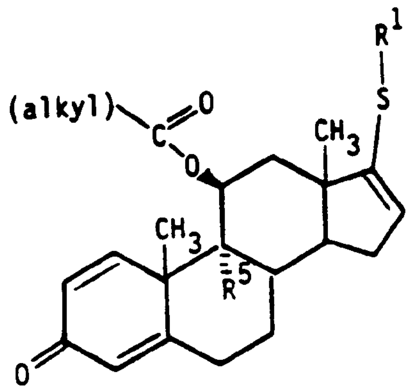

where

$R^1$ is alkyl; and

$R^5$ is halogen, especially fluoro,

comprising the step of contacting a 3-keto,17-thioketalandrostene of the formula Va:

(Va)

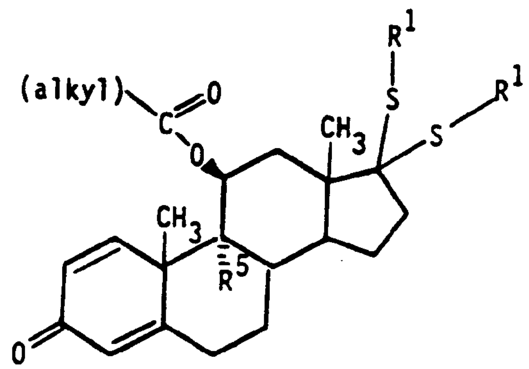

where $R^1$ and $R^5$ are as defined above for formula IVa and both $R^1$ groups are the same, with a protic acid catalyst.

The starting 3-keto,17-thioketalandrostenes of the formula V may be obtained by any suitable method. For example, the methods of U.S. Patent No. 4,361,559, which patent is incorporated herein by reference, may be employed. A preferred method of preparing the 3-keto-17-thioketalandrostene starting material is the inventive selective thioketalization process described above.

It is preferred to prepare the thioketal starting material in a separate step prior to the preparation of the thioenol ether according to the instant process.

The temperature employed in the instant process may be selected from those temperatures at which the reaction proceeds and the desired thioenol ether product is obtained. Preferred reaction temperatures are those from about 70°C to about 250°C, particularly from about 90°C to about 140°C. The temperature employed is preferably the reflux temperature of the solvent, although it is desirable to avoid temperatures causing product degradation. Use of the reflux temperature and continuous distillation of the solvent may facilitate the removal of any moisture present.

The process of the instant invention, by employing an acid catalyst, allows the same rate of reaction to be achieved at a lower temperature than the process conducted in the absence of the catalyst. The capability of conducting the reaction at a relatively low temperature provides a number of advantages. In particular, the degradation of product which may be observed at higher temperatures may be minimized or eliminated. Further, the use of a relatively low temperature provides an advantage in terms of safety, especially at larger scales. For example, steam at a lower pressure may be employed for heating.

Any protic acid enhancing the reaction rate, as compared with the process conducted under the same conditions in the absence of the acid, may be employed as the catalyst of the instant invention. The protic acid

catalyst may be employed alone or in conjunction with one or more additional protic acid catalysts.

Strong protic acids are preferred as the acid catalyst of the instant invention. In particular, those acids having a pKa less than or equal to about 2.5, especially less than or equal to about 0.65, are preferred. pKa may be determined, for example, as discussed in March, Advanced Organic Chemistry (1985). The strong protic acid may be employed directly or may be formed in situ.

Exemplary acids which may be employed include phosphoric acid, trihaloacetic acids such as trichloroacetic acid (preferred amounts are from about 0.5 to about 1.0 equivalent or less) or trifluoroacetic acid, pentafluoropropionic acid, organic sulfonic acids such as those of the formula R-$SO_3H$ where R is alkyl or aryl, including methanesulfonic acid (preferred amounts are about 0.05 equivalent or less) and p-toluenesulfonic acid, and, preferably, Lewis acid/protic source complexes.

It is preferred to employ input materials which are substantially, preferably completely, free of water to minimize or eliminate hydrolysis of the thioenol ether product. Hydrolysis of the thioenol ether product may produce unwanted side-products, for example, by the following reaction:

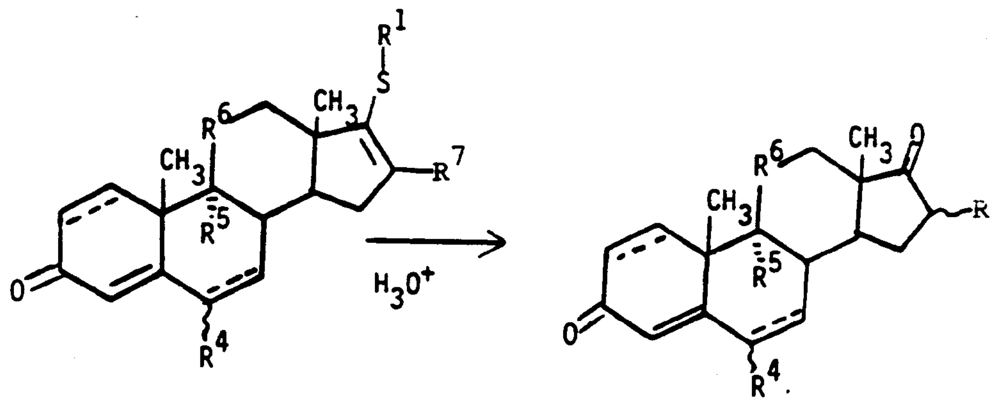


where the substituents are defined as above for the compounds of the formula IV. Free water may be removed, for example, by distillation.

In view of the potential for hydrolysis, and the difficulties which may be encountered in maintaining the input materials in a rigorously dry state, it is preferred to employ a protic acid catalyst, preferably a strong protic acid catalyst, which may be formed by the combination of a protic acid catalyst precursor with a protic source such as water. Such combination, in addition to providing the protic acid catalyst of the invention, may remove water present in the input components from being an effective agent in the hydrolysis of the thioenol ether product. Exemplary precursors include protic acid anhydrides such as trihaloacetic acid anhydrides, or, preferably, the Lewis acid of a Lewis acid/protic source complex. The protic acid catalyst may, for example, be formed in situ, or by contact of the precursor with one or more of the input components to remove residual moisture before contact with the remaining reactants.

Addition of free protic acid when the anhydride thereof is added, or an additional protic source when a Lewis acid is added, preferably one with less affinity for the Lewis acid than water, is desirable to ensure a sufficient quantity of protic acid catalyst to the extent that moisture is absent from the input components.

As discussed above, the preferred catalysts of the instant invention are Lewis acid/protic source complexes. Exemplary Lewis acids which may be employed include boron trifluoride or, especially, etherates thereof such as boron trifluoride ethyl etherate ($BF_3Et_2O$), as well as other Lewis acids such as $TiCl_4$, $SnCl_4$, $BBr_3$, $BCl_3$ and the like. Exemplary protic sources which may be employed include oxygen nucleophiles bearing an acidic proton such as water, organic alcohols, particularly alkanols such as methyl or ethyl alcohol, or organic protic acids such as organic carboxylic acids, particularly alkyl carboxylic acids such as acetic acid or trichloroacetic acid, alone or in combination with the anhydride thereof. Lewis acids capable of complexation with water are particularly preferred in view of the basicity of water, and since complexation removes water as an effective hydrolysis agent. Catalysts of this the may, of course, be added directly as well as formed in situ. Preferably, the protic source employed is other than a thiol having the structure of the thiol released during the reaction.

An exemplary such system is that formed by the addition of boron trifluoride ethyl etherate ($BF_3Et_2O$) and acetic acid (AcOH), where water present preferentially complexes with $BF_3Et_2O$. This system is illustrated as follows:

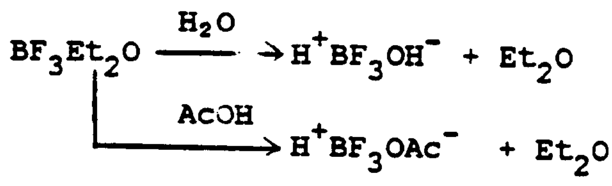


The $H^+BF_3OH^-$ and $H^+BF_3OAc^-$ complexes so formed are both strong protic acids which catalyze the instant reaction.

The amount of protic acid catalyst employed is that which is effective to catalyze the instant process. Preferred amounts are selected so that the molar ratio of acid catalyst, or, for example, Lewis acid when a Lewis acid/protic source complex is employed as the catalyst and the Lewis acid is the limiting component in forming the complex, to the 3-keto,17-thioketalandrostene starting material is from about 0.01:1 to about 0.2:1, particularly from about 0.03:1 to about 0.06:1, most preferably about 0.05:1. In general, it is desirable to increase the amount of catalyst employed as the reaction temperature is decreased.

A further preferred embodiment of the instant invention is that where the reaction rate is controlled so that the function of product concentration versus time is closer to linear than in the absence of the control. Control of the reaction rate according to this embodiment minimizes or eliminates the formation of side-products during the initial stages of an uncontrolled reaction. Such side-products may be formed for example, through reaction of the desired product with thiol released from the thioketal starting material as follows:

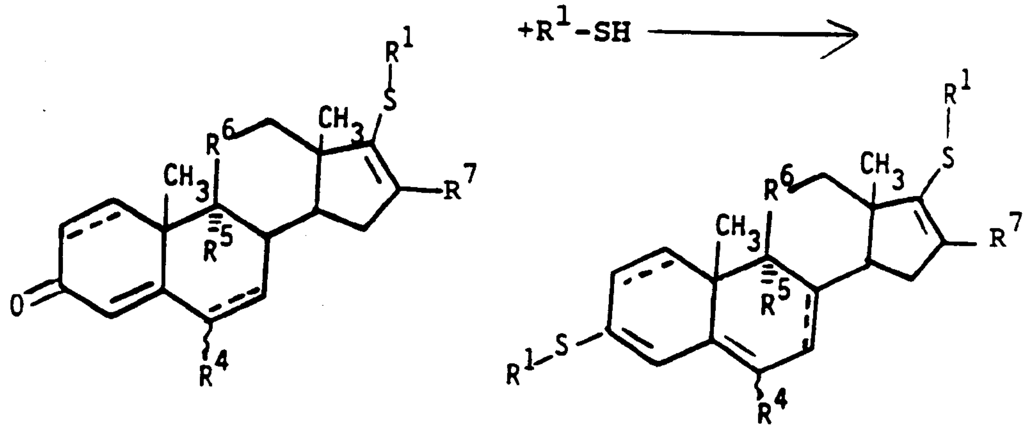


where the substituents are defined as above for the compounds of the formula IV.

Control of the reaction rate, particularly the initial reaction rate, is preferably achieved by control of the rate of catalyst addition, such as by addition at a gradually increasing rate. For example, it is preferred to add approximately one-third of the acid catalyst over the course of about the first two hours of the reaction. The remaining two-thirds of the catalyst may ten be added at once, or in large increments through the completion of the reaction. The controlled addition of the catalyst may be performed either by continuous or stepped addition. The catalyst may be added neat or, preferably, in a solvent such as that employed in the reaction medium.

In addition to controlling the initial reaction rate as discussed above, the formation of side-products may be minimized or eliminated by removal, during the reaction, of at least part of the thiol released from the thioketal starting material. Preferably, substantially all of the thiol formed is removed during the reaction. Thus, for example, the reaction show above is suppressed due to a deficiency of the $R^1$-SH thiol reactant.

Removal of the thiol is preferably achieved by co-distillation of the thiol with a reaction solvent. Additional solvent, preferably dry, may be added as desired to maintain the reaction volume. Such addition of solvent may be stepped or continuous. Removal of the thiol formed may also be facilitated by the use of a purge gas, preferably an inert gas such as nitrogen.

A solvent is preferably employed in the instant process. Exemplary solvents include inert, organic liquids such as toluene, xylenes, diethylbenzenes, dichlorobenzenes, or methyl isobutyl ketone, as well as mixtures thereof. The amount of solvent is preferably selected so that the loading of 3-keto,17-thioketalandrostene starting material is from about 5 to about 20 weight %, based upon the combined weight of the solvent and starting material.

The pressure and nature of the atmosphere employed may be selected by the skilled artisan.

The reaction is preferably terminated when the 3-keto,17-thioketalandrostene starting material is substantially consumed. Termination may be accomplished by neutralization of the acid catalyst, such as by the addition of triethylamine. The reaction mixture is then preferably cooled, such as to a temperature of approximately 90°C, and a liquid such as water added to extract the neutralized catalyst. Most of the water added may then be removed by allowing phase separation with subsequent removal of the aqueous layer. Residual moisture may be removed by a method such as azeotropic distillation of the reaction medium, with volume adjustment during distillation as appropriate. Crystallization of the product may be achieved, for example, by cooling and/or by the addition of a liquid in which the product is insoluble such as heptane, with concomitant agitation. The product may then be separated from the reaction medium by filtration, and dried, for example, under vacuum at 50 to 60°C.

The product may optionally be recrystallized to remove any residual impurities. For example, the product may be redissolved with triethylamine in hot isopropanol, with agitation, optionally with the use of activated carbon, and then concentrated and filtered to provide recrystallization. A liquid such as water may be added, and/or the slurry cooled, to facilitate recrystallization The recrystallized product may be filtered and dried as above.

Use of a protic acid catalyst according to the process of the instant invention, by providing an enhanced reaction rate, achieves greater efficiency in conducting the reaction at a given temperature. Further, by selecting process conditions as described above, a product with higher purity may be obtained, allowing simplified product isolation and purification steps to be employed. The reaction conditions are most preferably selected when conducting the instant process so as to prep are a compound of the formula IV substantially free of unwanted side-products.

The preferred methods for preparing 3-keto,17-thioenoletherandrostenes described herein may be employed as appropriate for the preparation of any thioenolether according to the instant process.

Preparation of Thioketals

The instant invention provides a process for the preparation of a thioketal from a thioenolether, comprising the step of contacting the thioenolether with a thiol in the presence of a protic acid catalyst. In a particularly preferred embodiment, the instant invention provides a process for the preparation of a mixed 3-keto,17-thioketalandrostene of the formula VI:

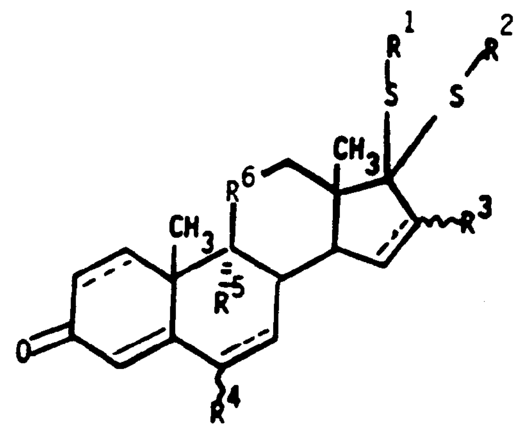

(VI)

where

$R^1$ and $R^2$ are different, and are alkyl, cycloalkyl or aryl;

$R^3$ is hydrogen, alkoxy, aryloxy, alkylthio, arylthio, alkylcarbonyloxy or halogen or, when the 15,16-positions are saturated, hydroxy;

$R^4$ is hydrogen, alkyl, hydroxy, alkylcarbonyloxy or halogen;

$R^5$ is hydrogen or halogen; and

$R^6$ is carbonyl, (β-hydroxy)methine or protected (β-hydroxy)methine,

comprising the step of contacting a 3-keto,17-thioenoletherandrostene of the formula IV:

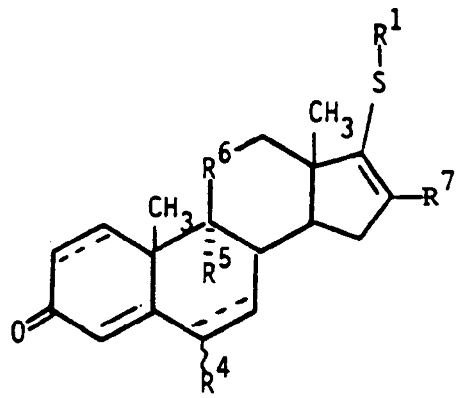

(IV)

where

$R^7$ is hydrogen, alkoxy, aryloxy, alkylthio, arylthio, alkylcarbonyloxy or halogen; and

$R^1$, $R^4$, $R^5$ and $R^6$ are as defined above for the formula VI,

with a thiol of the formula VII:

$R^2$-SH (VII)

where $R^2$ is as defined above for the formula VI in the presence of a protic acid catalyst.

It is particularly preferred to prepare, according to the process of the instant invention, those compounds of the formula VI which are $\Delta^{1,4}$-androstadienes in which:

$R^1$ and $R^2$ are different, and are alkyl, especially compounds where $R^1$ is β-methyl and $R^2$ is α-ethyl;

$R^3$ and $R^4$ are hydrogen;

$R^5$ is halogen, especially fluoro; and

$R^6$ is protected (β-hydroxy)methine, especially alkylcarbonyloxymethine such as acetyloxymethine, which is subsequently deprotected.

Thus, an especially preferred method of the instant invention relates to a process for the preparation of a mixed 3-keto,17-thioketalandrostene of the formula VIa:

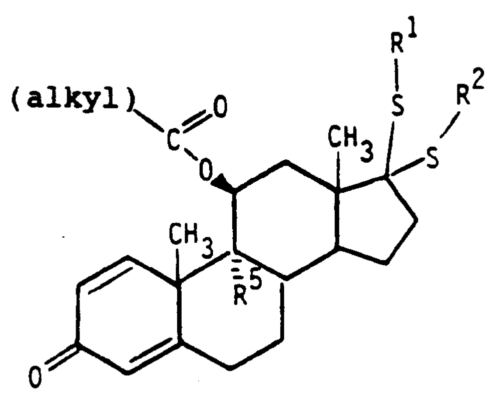

(VIa)

where

$R^1$ and $R^2$ are different, and are alkyl; and

$R^5$ is halogen,

comprising the step of contacting a 3-keto,17-thioenoletherandrostene of the formula IVa:

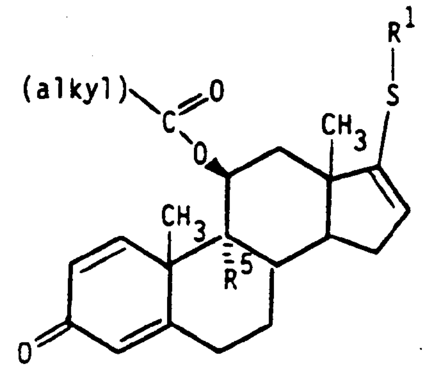

(IVa)

where $R^1$ and $R^5$ are as defined above for the formula VIa with a thiol of the formula VIIa:

$R^2$-SH (VIIa)

where $R^2$ is as defined above for the formula VIa; in the presence of a protic acid catalyst.

The starting 3-keto,17-thioenoletherandrostenes of the formula IV may be obtained by any suitable method. For example, the methods of U.S. Patent No. 4,361,559, incorporated herein by reference, may be employed. Preferably, the methods for preparing thioenol ethers from thioketals described above are employed, particularly in which a 3,17-diketoandrostene may be converted to a 3-keto,17-thioketalandrostene where both thiol groups at the 17-position are the same, and where the latter compound is then converted to a 3-keto,17-thioenoletherandrostene.

For preparing the compounds of formula VI of the instant invention, thiols of the formula VII are readily obtainable by those skilled in the art. Preferred amounts of thiol in the instant process are those providing a molar ratio of thiol: 3-keto,17-thioenoletherandrostene starting material of from about 2:1 to about 10:1, particularly from about 4:1 to about 6:1. Higher concentrations of thiol facilitate more rapid conversion of the formula IV compounds to those of the formula VI,and shift the equilibrium of the instant reaction to enhance formation of the desired product.

Any protic acid enhancing the reaction rate, as compared with the process conducted under the same conditions in the absence of the protic acid, may be employed as the catalyst of the instant invention. The protic acid catalyst may be employed alone or in conjunction with one or more additional protic acid catalysts. Preferred amounts of protic acid catalyst are those providing, in the reaction medium, a molar ratio of acid catalyst:3-keto,17-thioenoletherandrostene starting material of from about 0:1 to about 8:1, particularly from about 1:1 to about 5:1. Higher concentrations of catalyst allow more rapid reaction rates. When a Lewis acid/protic source complex as discussed below is employed as the protic acid catalyst, the molar ratio may be calculated on the basis of Lewis acid:starting material when the Lewis acid is the limiting component in forming the complex.

Use of a catalyst which is a protic acid, according to the instant invention, allows shorter reaction times to be employed. Exemplary protic acid catalysts include trihaloacetic acids such as trichloroacetic acid or trifluoroacetic acid, pentafluoropropionic acid, phosphoric acid, organic sulfonic acids such as those of the formula $R-SO_3H$ where R is alkyl or aryl, for example, methanesulfonic acid and p-toluenesulfonic acid, and Lewis acid/protic source complexes. Exemplary Lewis acids which may be employed in the aforementioned complexes include boron trifluoride or etherates thereof such as boron trifluoride ethyl etherate ($BF_3Et_2O$), as well as other Lewis acids such as $SnCl_4$, $TiCl_4$, $BBr_3$, $BCl_3$ and the like. Exemplary protic sources which may be employed include oxygen nucleophiles bearing an acidic proton such as water, organic alcohols, particularly alkanols such as methyl or ethyl alcohol, and organic protic acids such as organic carboxylic acids, especially alkyl carboxylic acids such as acetic acid and trifluoroacetic acid, alone or in combination with the anhydride thereof. As used herein, the "protic source" employed for the conversion of a thioenol ether to a thioketal may not consist of the thiol reactant when boron trifluoride ethyl etherate is used as the Lewis acid. It is also preferred, for other Lewis acids, that the protic source employed not consist of the thiol reactant. Further, it is preferred that water, such as trace water, not be the sole protic source employed, for example, when boron trifluoride ethyl etherate is employed.

To minimize or eliminate hydrolysis of the thioenol ether starting material of the formula IV, it is preferred to employ input materials which are substantially, preferably completely, free of water. In this regard, it is further preferred to employ a precurser of the protic acid catalyst which, in combination with water, provides the protic acid catalyst of the inventive process while removing water as an effective hydrolyzing agent. Exemplary precursors include protic acid anhydrides such as trihaloacetic acid anhydrides, or the Lewis acid of a Lewis

acid/protic source complex. Addition of free protic acid when the anhydride thereof is added, or an additional protic source when a Lewis acid is added, preferably one with less affinity for the Lewis acid than water, is desirable to ensure a sufficient quantity of protic acid catalyst to the extent that moisture is absent from the input components.

When employing a trihaloacetic acid, it is preferred that the acid be pre-treated, such as by pre-heating, with the anhydride thereof, to remove residual moisture before contact with the other reactants. Preferred molar ratios of acid:anhydride for such pre-treatment are from about 50:1 to about 3:1, especially about 9:1.

A solvent is preferably employed in the reaction medium. Exemplary solvents include inert, organic liquids such as dimethylformamide, N-methyl pyrrolidone, toluene, xylenes or halogenated hydrocarbons such as dichlorobenzenes, or, especially, methylene chloride, as well as mixtures thereof. Dimethylformamide and N-methyl pyrrolidone are preferred solvents when $R^6$ of the formula IV starting material is (β-hydroxy)methine, as such compounds may have lower solubilities relative to those compounds where $R^6$ is protected (β-hydroxy)methine. Preferred amounts of solvent are those where the loading of the 3-keto,17-thioenoletherandrostene starting material is from about 1 to about 15 weight % based on the combined weight of the starting material and solvent.

The pressure and nature of the atmosphere employed may be selected by the skilled artisan.

A preferred order of addition for contacting the components of the instant process is that where the 3-keto,17-thioenoletherandrostene starting material is added to, and is preferably completely or substantially completely dissolved in, a solvent with subsequent addition of thiol, and then acid catalyst.

The mixed 3-keto,17-thioketalandrostene products of the instant process are chiral substances at the 17-position. Thus, stereoisomers may be formed as follows:

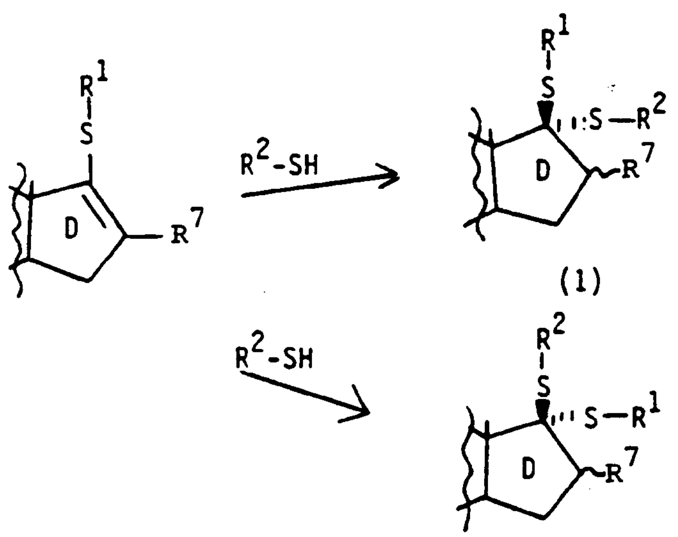

(1)

(2)

Whether stereoisomer (1) is formed preferentially relative to stereoisomer (2) is directly a function of temperature and, indirectly, a function of the nature of the catalyst employed. In this regard, lower temperatures facilitate the addition of thiol in a manner wherein the stereoisomer (1) is preferentially formed. Thus, to obtain the stereoisomer (1) while minimizing the formation of the stereoisomer (2), the reaction temperature may be lowered, for example, to a temperature less than about 20°C, particularly to a temperature of from about -70 to about 10°C.

The use of certain preferred protic acid catalysts may further reduce the formation of stereoisomer (2) at any given temperature by suppressing the "scrambling" reactions discussed below. By the use of a preferred protic acid catalyst of the instant invention, therefore, the same degree of stereospecificity may be achieved at a relatively higher temperature, due to enhanced suppression of "scrambling" reactions, as may be achieved at a lower temperature with a less preferred catalyst. This is particularly advantageous since, especially at larger scales, it may be desirable to employ relatively high reaction temperatures, preferably those from about -25 to about 0°C, which are more readily attained from a practical standpoint.

The "scrambling" reactions referred to above result due to the reversible nature of the reaction forming the mixed 3-keto,17-thioketalandrostene product. Thus, there is a tendency to form a number of side-products, for example, by reversal of the desired reaction:

(A)

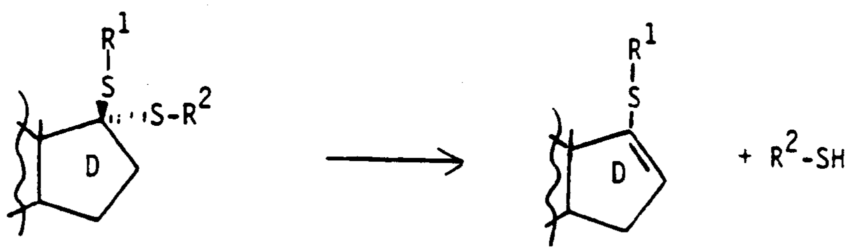

or by elimination of the -S-$R^1$ thioether group from the desired product:

(B)

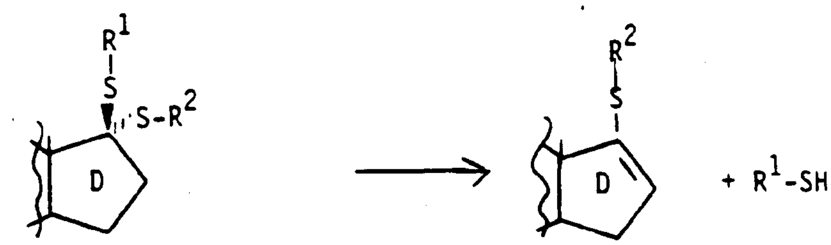

These side-products may themselves further react to form additional side-products, for example, by addition of the available thiol $R^2$-SH to the side-product previously formed by elimination of the -S-$R^1$ thioether group in reaction B above:

(C)

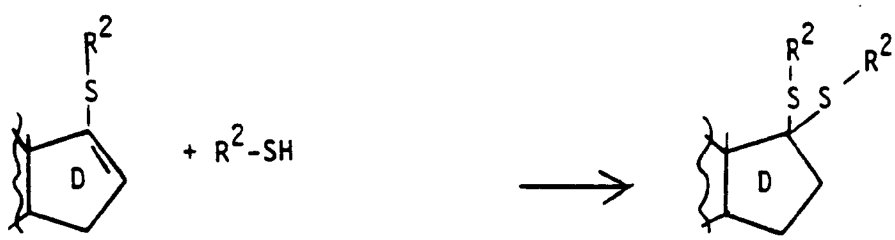

or by addition of an undesired thiol, $R^1$-SH, to the starting material:

(D)

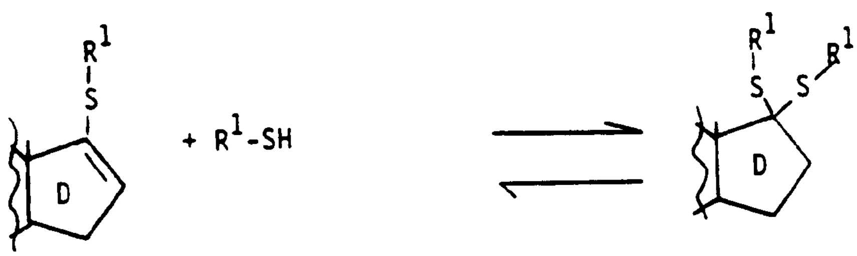

The thiol $R^1$-SH, a reactant in reaction D above, may be present as a result of the elimination of the -S-$R^1$ thioether group from the desired product in the above reaction B, or by hydrolysis of the starting material, as follows:

(E)

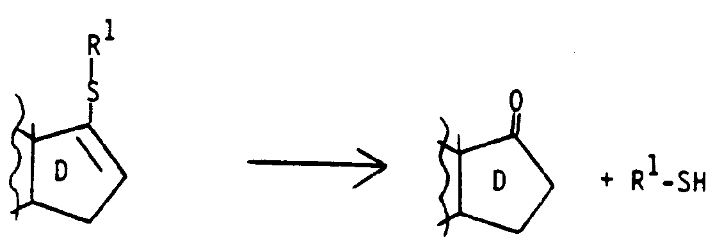

Further, stereoisomer (2) may be formed by addition of the thiol $R^1$-SH to the thioenolether formed in reaction B above, as follows:

(F)

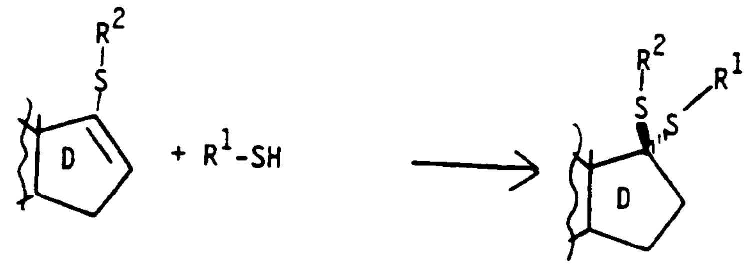

Suppressing the above "scrambling" reactions thus not only enhances preferential preparation of stereoisomer (1), but also minimizes or eliminates the formation of other side-products such as those formed in the above reactions.

A preferred embodiment of the instant invention thus provides a process for the preparation of a mixed 3-keto,17-thioketalandrostene, with a high degree of stereospecificity and high purity, at higher, as well as at lower temperatures, by the use of a protic acid catalyst having a greater, that is, a more positive, $pK_a$. (pKa may be determined as discussed in March, Advanced Organic Chemistry (1985)). In particular, such results may be achieved by the use of a protic acid catalyst having a $pK_a$ less than or equal to that $pK_a$ at which the 3-keto,17-thioenoletherandrostene starting material will first accept and become activated by a proton, and greater than or equal to about -10, especially a protic acid catalyst having a $pK_a$ of from about 2.5 to about -4. Those acid catalysts having pKas at the higher ends of these ranges provide greater control of the reaction rate, and thus are especially preferred.

In this regard, trihaloacetic acids, particularly trichloroacetic acid, are preferred. The use of trichloroacetic acid also facilitates dissolution of the thioenolether starting materials, where $R^6$ is unprotected (β-hydroxy)methine, over a wide range of reaction temperatures.

Use of the preferred protic acid catalysts according to the instant invention allows improved control over reaction times and temperatures, as well as minimizing the formation of side-products including unwanted stereoisomers. The invention, therefore, also allows simplified product isolation and purification steps to be employed.

A general procedure for isolation and purification of the mixed 3-keto,17-thioketalandrostenes of the formula VI is that where a base such as sodium hydroxide is added to neutralize and extract the catalyst employed, as well as any excess thiol. Trace metals are preferably removed to aid in preventing a reversal of the desired reaction, such as by the addition of an ethylenediamine tetraacetic acid (EDTA)/triethylamine (TEA) mixture.

When the instant process is conducted with a milder protic acid at a higher temperature, a simplified quench procedure may be employed. Thus, while a quench liquid such as sodium carbonate in a water/methanol solution may be employed at lower temperatures, aqueous sodium hydroxide, for example, a IN aqueous sodium hydroxide solution, may be employed to quench a reaction conducted at higher temperatures. The greater base strength of sodium hydroxide provides, for example, a more thorough extraction of excess thiol.

Isolated product may be purified, if desired, by recrystallization. A preferred recrystallization method is that where the crude product is dissolved in a solvent such as hot isopropanol, optionally with activated carbon, filtered, and is then crystallized, for example, by cooling and/or by the addition of a nonsolvent such as water.

Addition of a hydroxyl group at the 16-position of a compound of the formulaVI, where the 15,16-positions of the compound are saturated, may be achieved by methods known to the skilled artisan.

The preferred methods for preparing mixed 3-keto,17-thioketalandrostenes described herein may be employed as appropriate for the preparation of any thioketal, particularly any mixed thioketal, according to the instant process.

Those compounds of the formula I where $R^6$ is carbonyl or (β-hydroxy)methine are particularly useful as antiinflammatory agents. Most particularly, compounds of the formula VI in which $R^6$ is carbonyl or (β-hydroxy)methine are useful as antiinflammatory agents. Those compounds of the formula VI in which $R^6$ is protected (β-hydroxy)methine are therefore preferably deprotected, such as by known methods, to provide compounds in which $R^6$ is (β-hydroxy)methine. Deprotection may also facilitate the crystallization of the 11-hydroxyl product. Deprotection of an alkylcarbonyloxy protecting group, to yield a hydroxyl group at the 11-position, may be accomplished, for example, by solvolysis with a base such as sodium hydroxide in a medium, preferably heated, such as an aqueous alcohol, for example, methanol and water, solution. The base may subsequently be neutralized by the addition of an acid such as acetic acid.

The compounds of the formulas I and VI useful as antiinflammatory agents may be employed, for example, in the treatment of inflammatory conditions in mammalian hosts. Thus, they may be used as topical antiinflammatory agents, for example, in the treatment of skin conditions such as dermatitis, psoriasis, sunburn, eczema, neurodermatitis or anogenital pruritis, and in inhalation therapy for the topical treatment of allergy and asthma.

For the treatment of skin conditions. the compounds of the formulas I and VI may be administered in a conventional pharmaceutical carrier in the form of a cream, ointment, lotion or the like. These compounds will preferably be used in the range of from about 0.01 to about 5.0% by weight of the vehicle, most preferably from about 0.05 to about 2.0% by weight of the vehicle.

For the topical treatment of allergy and asthma the compounds of the formulas I and VI may be administered in any known manner, such as by use as a solid medicament which has been atomized. Any known device useful for administration of solid medicaments for inhalation therapy may be employed. Dosages may be selected by the skilled artisan according to known procedures.

Those compounds of the formula I where $R^6$ is protected (β-hydroxy)methine may be deprotected by means known to those skilled in the art to form compounds of the formula I useful as an antiinflammatory agents as discussed above. Further, any of the compounds of the formula I, that is, compounds where $R^6$ is carbonyl, (β-hydroxy)methine or protected (β-hydroxy)methine, may be converted to mixed thioketals of the formula VI, such as where $R^6$ is carbonyl or (β-hydroxy)methine. The mixed thioketals of the formula VI are preferred as antiinflammatory agents. Particularly preferred compounds of the formula VI are $\Delta^{1,4}$-androstadienes where $R^1$ and $R^2$ are alkyl, especially such compound in which $R^1$ is β-methyl and $R^2$ is α-ethyl, $R^3$ and $R^4$ are hydrogen, $R^5$ is fluoro, and $R^6$ is (β-hydroxy)methine.

A compound of formula I may be converted to a compound of the formula VI by any suitable method. For example, the methods of U.S. Patent No. 4,361,559, incorporated herein by reference, may be employed. Preferably, one or more of the methods described above may be employed. Thus, the instant invention provides a process for the preparation of a mixed 3-keto,17-thioketalandrostene of the formula VI, such as where $R^6$ is carbonyl or (β-hydroxy)methine, comprising the steps of:

(a) preparing a 3-keto,17-thioketalandrostene of the formula I by a process comprising the step of contacting a 3,17-diketoandrostene of the formula II with a thiol of the formula III, in the presence of a protic acid catalyst which is a complex of a Lewis acid and a protic source, wherein the temperature of the reaction medium is maintained below about 25°C during the reaction, and, preferably, where (i) the acid catalyst is employed in excess, and/or (ii) the thiol is employed in excess; and

(b) converting the 3-keto,17-thioketalandrostene of the formula I prepared in step (a) to the mixed 3-keto,17-thioketalandrostene of the formula VI.

The compounds of the formula IV are useful as intermediates in the preparation of mixed 3-keto,17-thioketalandrostene antiinflammatory agents of the formula VI, where $R^6$ is carbonyl or (β-hydroxy)methine, particularly the preferred compounds of formula VI described above.

A compound of the formula IV may be converted to a compound of the formula VI by any suitable method. For example, the methods of U.S. Patent No. 4,361,559, incorporated herein by reference, may be employed. Preferably, the methods described above are employed.

Thus, the instant invention provides a process for the preparation of a mixed 3-keto,17-thioketalandrostene of the formula VI, such as where $R^6$ is carbonyl or (β-hydroxy)methine, comprising the steps of:

(a) preparing a 3-keto,17-thioenoletherandrostene of the formula IV by a process comprising the step of contacting a 3-keto,17-thioketalandrostene of the formula V with a protic acid catalyst; and

(b) converting the 3-keto,17-thioenoletherandrostene of the formula IV prepared in step (a) to the mixed 3-keto,17-thioketalandrostene of the formula VI.

The 3-keto,17-thioketalandrostene of formula V of this process is preferably prepared from the corresponding 3,17-diketoandrostene according to above-described selective thioketalization process of the present invention.

The following examples are illustrative only, and are not intended to limit the scope of the instant claims.

EXAMPLE 1

Preparation of (11β)-9-Fluoro-11-hydroxy-17,17-bis(methylthio)androsta-1,4-dien-3-one

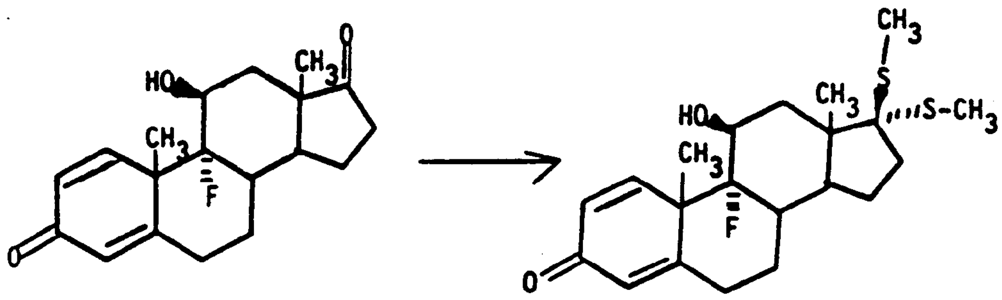

Glacial acetic acid (45.0 L) was transferred to a 400 L stainless-steel reactor, jacketed for heating and cooling, and equipped with a condenser, and agitation begun. (11β)-9-Fluoro-11-hydroxyandrosta-1,4-dien-3,17-dione (10.0 kg) was then transferred to the reactor while agitation was continued. Most of the starting material remaine undissolved at this point. The temperature of the slurry was lowered to between 16 and 18°C. Methanethiol (8.3 kg), chilled to less than -20°C, was added to the reactor under slight nitrogen pressure.

The mixture was then agitated until all the starting material dissolved. During this time, a slight temperature elevation to enhance the dissolution rate was allowed, not exceeding +17°C, with cooling begun immediately after full dissolution was achieved to avoid unnecessary loss of thiol. The temperature of the solution in the reactor was lowered to about +4°C. Dry borontrifluoride ethyl etherate (19.3 L, 5.1 gallons) at a temperature between -50 and -55°C was transferred to the reactor to start the reaction. Rapid addition and dispersal of the catalyst into the mixture, within 10 to 20 seconds, was conducted.

After four minutes had elapsed from the start of catalyst addition, during which time the maximum temperature obtained was about 5°C, deionized water (approximately 66 to 70 L), chilled to a temperature between 1 and 3°C, was added within 60 to 90 seconds to quench the reaction. Essentially all steroidal material was precipitated by this action, although crystallization of most of the product occurred prior to quenching. The temperature of the mixture rose by about 10°C following the quench, which temperature increase was minimized by pre-chilling of the water added. With continued agitation, the batch temperature was lowered to 1 to 4°C, and this temperature maintained for no less than 30 minutes. The product was transferred to a filter, separating the crude product from the filtrate. Deionized water (approximately 140 to 148 L), chilled to a temperature between 1 and 3°C, was also passed through the reactor and the filter, transferring any residual product to the filter and washing the product.

The crude product was then transferred from the filter, using 5 to 15 L of isopropanol as a wash, into about 35 L of isopropanol, providing a total volume of 40 to 50 L of isopropanol. With agitation, the isopropanol slurry was heated to near-reflux (78 to 82°C), and this temperature maintained for 10 to 15 minutes. The slurry was cooled to between 20 and 25°C, and this temperature maintained with continued agitation for no less than 45 minutes. The isopropanol slurry was transferred to the filter, separating the purified product, and the mother liquor recycled until clear. The vessel employed for the isopropanol slurry was agitated with isopropanol (40 L), and this liquid was also passed through the filter, transferring any product remaining and washing the purified product. The product was transferred to a dryer, and a temperature of no greater than 50°C was maintained under high vacuum (about 20 mm Hg) until constant weight was achieved (within 10 to 12 hours). A yield of 96 mole % of the title product was obtained.

EXAMPLE 2

Compounds of the formula I as described in the following Table 1 are prepared by employing a procedure analogous to that of Example 1 above, where the thiol and/or starting material is substituted as indicated.

TABLE 1

| Starting Material | Thiol | Formula I Compound |
|---|---|---|
| 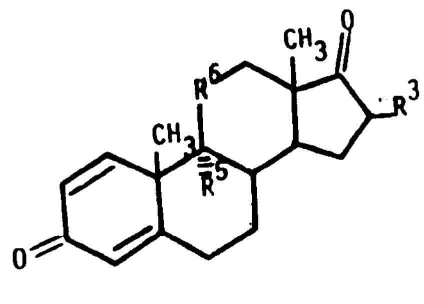 | $R^1$-SH | 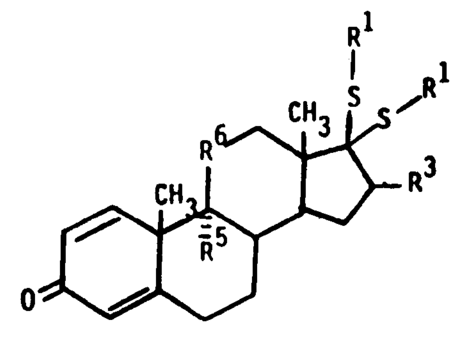 |

| | $R^3$ | $R^5$ | $R^6$ | $R^1$ |
|---|---|---|---|---|
| (a) | OH | F | β-HO-CH | $CH_3$ |
| (b) | H | H | β-HO-CH | $C_2H_5$ |
| (c) | H | F | β-HO-CH | n-$C_3H_7$ |
| (d) | H | F | β-HO-CH | n-$C_4H_9$ |
| (e) | $CH_3O$ | F | β-HO-CH | $C_2H_5$ |
| (f) | H | F | β-HO-CH | $C_6H_5$ |
| (g) | H | F | β-HO-CH | $C_2H_5$ |
| (h) | H | F | β-HO-CH | p-$CH_3O$-$C_6H_5$ |
| (i) | H | F | β-$CH_3COO$-CH | $CH_3$ |
| (j) | H | F | β-$CH_3COO$-CH | $C_2H_5$ |
| (k) | H | F | β-$CH_3COO$-CH | $C_6H_5$ |
| (l) | H | H | β-HO-CH | $CH_3$ |

Example 3

Preparation of (11β)-11-(Acetyloxy)-9-fluoro-17-(methylthio)androsta-1,4,16-triene-3-one

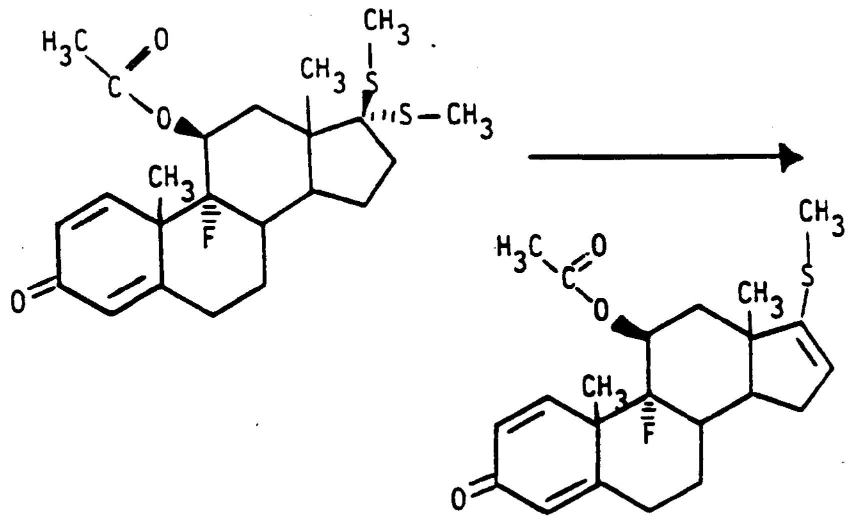

Toluene (approximately 113 L) was added to a 450 L glass-lined reactor, jacketed for heating and cooling and equipped with a condenser, and agitation begun. (11β)-11-(Acetyloxy)-9-fluoro-17,17-bis(methylthio)androsta-1,4-dien-3-one (10.00 kg) was then added, and the mixture heated to the distilling temperature (about 110°C). The starting material dissolved completely with the rise in temperature.

Toluene was distilled from the reactor until a batch volume of approximately 90 L was achieved. The solution was checked for moisture, and distillation repeated, with addition of fresh toluene, until a moisture level of 0.01% or less was obtained.

Dry toluene (18 L, moisture level < 0.01%) was added to a thoroughly pre-dried catalyst supply vessel, to which was added glacial acetic acid (78 mL) and borontrifluoride ethyl etherate (168 mL). This solution was mixed well.

With venting to a scrubber, a 15 to 25 L/min nitrogen purge of the reactor headspace was begun. During the reaction, the stream of nitrogen would serve to sweep approximately 1.1 kg of methanethiol formed out of the reaction vessel, and was continued until the addition of triethylamine as discussed below.

The batch temperature was kept at least at 110°C. A slow addition of catalyst was begun to the mixture. About one-third of the total catalyst solution was added steadily over the first 2 hours; the remainder was added at a faster rate over the remaining 2 to 3 hours. A slow but steady rate of toluene distillation was continued, while making up the volume loss with fresh, dry toluene.

Within 4 hours of reaction time, the progress of the reaction was checked by HPLC. Continued monitoring of the reaction by HPLC was conducted to establish completion of the reaction (<0.3% starting material remaining). This occurred within 4 to 6 hours of total reaction time.

When the reaction was complete, the reaction mixture was cooled to between 100 to 105°C, and triethylamine (700 mL) was added to quench the catalyst present. The pH was checked and adjusted with additional triethylamine, as required, to achieve a pH greater than 7.

The batch was cooled to 80 to 85°C, and deionized water (6.7 L) slowly added. The batch was mixed for five minutes, and the phases were then allowed to separate.

The lower aqueous layer was withdrawn, and transferred to a glass gondola extraction vessel. This aqueous extract was cooled to below 35°C before also adding methylene chloride (up to 3.3 L) to the extraction vessel. The contents of the extraction vessel were thoroughly mixed, and after allowing the phases to settle, the methylene chloride layer was withdrawn, and slowly added back to the reactor.

The batch in the reactor was concentrated from an initial volume of approximately 90 L to approximately 45 L by distillation at atmospheric pressure. Distillation was continued when residual water remained in the mixture (as evidenced by the lack of a steady boiling point of about 110°C), with addition of toluene as necessary to maintain a volume of approximately 45 L, until all water was removed. The batch was cooled to a temperature between 100 to 105°C.

While vigorously agitating at a batch temperature of no less than 95°C, heptane (approximately 11 L, bp

98°C) previously held at room temperature was added. Crystallization started and proceeded smoothly, but rapidly, toward the end of this addition while agitation was continued. When necessary, the batch was allowed to cool slightly to start crystallization. Additional heptane (89 L) was added over no less than 30 minutes. During this addition, the slurry temperature gradually dropped.

The crystal slurry was cooled to between 0 to 5°C over 30 minutes or more, and vigorous agitation was maintained at this temperature for one or more additional hours. The crystal slurry was then transferred to a centrifuge with recycling of the mother liquor, when needed, to aid in the transfer.

The filter cake was washed in place with heptane (up to 17 L). The wet cake was transferred to a dryer, and maintained under vacuum (<20 mm Hg) at 50 to 60°C until constant weight was obtained, yielding the title product.

Two demonstration runs of the above process, each scaled to 16.5 kg input of steroidal starting material, yielded 13.0 kg (88%) and 13.2 kg (90%) of the title product.

Example 4

Compounds of the formula IV as described in the following Table 2 are prepared by employing a procedure analogous to that of Example 3 above, where the starting material is substituted as indicated.

TABLE 2

Starting Material

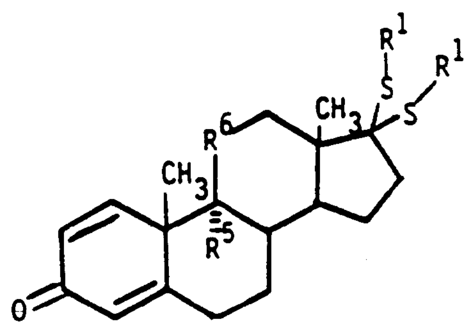

Formula IV Compound

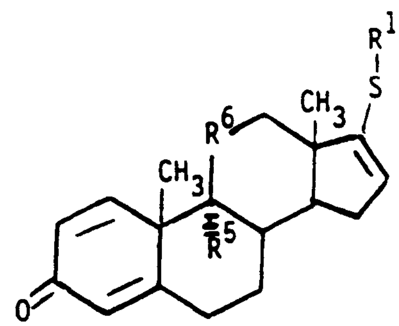

| | $R^1$ | $R^5$ | $R^6$ |
|---|---|---|---|
| (a) | $C_2H_5$ | F | β-$CH_3COO$-CH |
| (b) | $C_6H_5$ | F | β-$CH_3COO$-CH |
| (c) | $CH_3$ | H | β-OH |
| (d) | $C_2H_5$ | H | β-OH |

EXAMPLE 5

Preparation of (11β,17α)-11-(Acetyloxy)-17-(ethylthio)-9-fluoro-17-(methylthio)androsta-1,4-dien-3-one with $CCl_3COOH$ catalyst

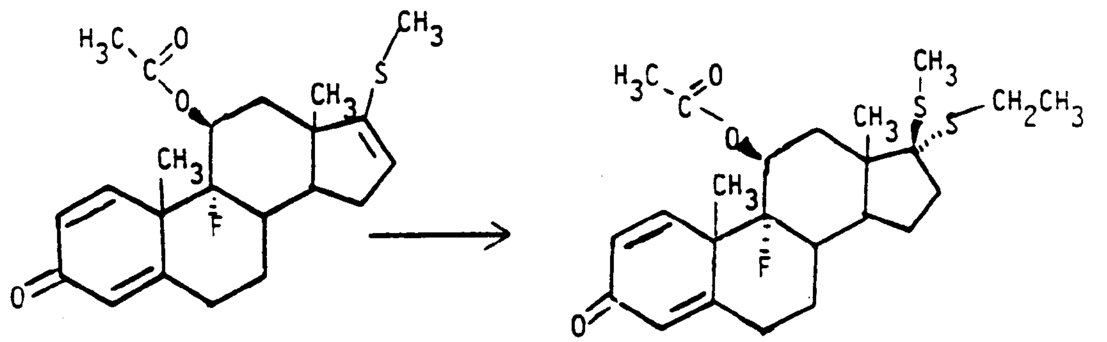

A catalyst mixture was prepared by moderate agitation of a mixture of methylene chloride (25 L), with the addition of additional methylene chloride as needed (up to 6 L) to obtain better agitation, trichloroacetic anhydride (0.94 L, 1.58 kg), and trichloroacetic acid (6.28 kg).

The mixture was warmed to or near gentle reflux (about 40°C) and maintained at this temperature for at least 30 minutes. Reflux was continued until samples showed that the solution contained no more than 0.02% water.

Methylene chloride (140 L) was then added to a 400 L stainless steel reactor, jacketed for heating and cooling, and equipped with a condenser. (11β)-11-(Acetyloxy)-9-fluoro-17-(methylthio)androsta-1,4,16-trien-3-one (10.00 kg) was added to the reactor, with mild agitation to effect dissolution. When the solution contained more than 0.02% moisture, methylene chloride (40 L) was added and distilled to about 145 L. This sequence was repeated as needed to dry the solution to below 0.02% moisture.

While maintaining a slight positive pressure in the reactor with nitrogen to insure dryness, the solution was cooled to between -20 to -24°C. The acid catalyst mixture prepared earlier was cooled to between -15 and -20°C.

Ethanethiol (7.95 kg, 9.5 L) was added to the reactor under mild agitation. The chilled catalyst mixture was then added to the reactor to start the reaction, and a batch temperature of between -18 to -22°C was maintained.

After 45 minutes, the progress of the reaction was checked by HPLC. As necessary, sampling was continued at additional 15-minute intervals until no more than 0.4% starting material remained unconverted to the title product. The reaction was completed within 2 hours.

A 1N (4 wt%) aqueous sodium hydroxide solution was prepared in a separate vessel by mixing 50% aqueous sodium hydroxide (approximately 25.6 kg) with deionized water to achieve a total volume of 320 L. 160 L of the 4 wt% sodium hydroxide solution at a temperature not greater than 30°C were added as rapidly as possible to the reactor, while maintaining vigorous agitation, to quench the reaction. Agitation was continued in the reactor for an additional 15 minutes. The phases were then allowed to settle, and the lower methylene chloride layer was transferred to a separate vessel for temporary holding.

Methylene chloride (25 L) was added to the reactor, the mixture was agitated vigorously for five minutes, and the phases were allowed to settle. The lower methylene chloride phase in the reactor was also transferred to the vessel holding the methylene chloride layer previously obtained.

The spent aqueous layer in the reactor was removed, and the reactor washed with deionized water (up to 10 L). The combined methylene chloride extracts were transferred from the holding vessel back to the reactor, using methylene chloride (up to 12 L) as a wash for the holding vessel.

1N (4 wt%) sodium hydroxide solution (160 L) was added to the reactor, and the mixture was agitated thoroughly for five to ten minutes. After allowing the phases to settle, the lower methylene chloride layer was transferred back to the holding vessel, and the back-extraction procedure above employing 25 L of methylene chloride was repeated.

An ethylenediamine tetraacetic acid/triethylamine (EDTA/TEA) solution was prepared by adding deionized water (668 mL), triethylamine (72 mL), and, with agitation, EDTA (38 g) to a flask, with mixing until all solids were dissolved. The EDTA/TEA solution was dispersed into methanol (16 L), mixed well, and the mixture was then added to the rich methylene chloride solution. This EDTA solution was added to sequester harmful trace metals.

The batch, mixed well and containing the title product, was held overnight and then employed directly in

the preparation of the title compound of Example 6.

EXAMPLE 6

Preparation of (11β,17α)-17-(Ethylthio)-9-fluoro-11-hydroxy-17-(methylthio)androsta-1,4-dien-3-one

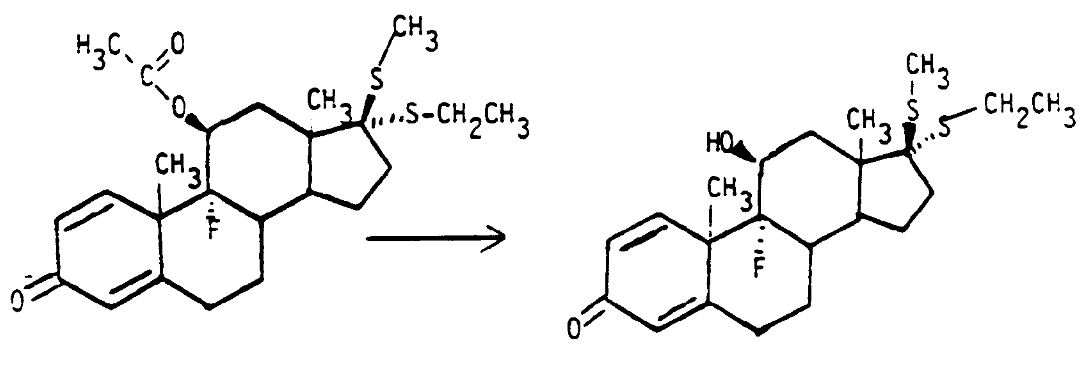

The methylene chloride extracts from Example 5 were concentrated by distillation under atmospheric pressure until a low, but easily agitated, volume was reached. Methanol was added while distillation was continued at constant volume until a minimum temperature of 60°C was attained. Additional methanol was added as necessary to bring the total volume to 200 L, and the batch was heated to near boiling (about 62°C) to fully dissolve any solids present.

A 5 N (20 wt%) aqueous sodium hydroxide solution was prepared by mixing 50% aqueous sodium hydroxide (4.1 kg) with deionized water to achieve a total volume of 10.2 L. The total volume of this solution was added while distilling methanol at atmospheric pressure. Within 5 minutes, crude title product began to crystallize from the boiling mixture.

After a total of 15 minutes from the time of base addition, completion of the reaction was confirmed by HPLC (<0.1% starting material remaining). The temperature of the batch was lowered slightly, and sufficient glacial acetic acid added to lower the pH to between 6 and 8 (approximately 1.8 Kg or 1.7 L).

Distillation of methanol was continued until a total volume of ca. 70 L was reached, and the addition of deionized water was begun to maintain the volume at about 70 L during continued distillation at atmospheric pressure.

Once a batch temperature of 90°C was reached, distillation was discontinued, and the volume of the batch adjusted to about 110 L through the addition of deionized water. After cooling to 0 to 5°C, the batch was transferred to a centrifuge, using a recycle of the filtrate, as needed, to aid in the transfer.

The crude title product was washed with room temperature deionized water (ca. 50 L). The product was transferred to a dryer and maintained at 50 to 60°C, under vacuum, until dried to constant weight. About 10.4 kg of crude title product was obtained (99%). Stereoselectivity, measured by a silver-catalyzed elimination assay, was established to be about 99.9% of the desired isomer.

To further enhance the purity of title product obtained, isopropanol (182 L) was transferred to a dissolution vessel, and agitation of the vessel contents begun.

Deionized water (1 L) was added to a 2 L laboratory flask, and triethylamine (TEA) (100 mL) was added. Ethylenediaminetetraacetic acid (EDTA) (51.5 g) was added with agitation, and mixed until all solids were dissolved. The EDTA/TEA solution so prepared was added to the vessel containing isopropanol to remove trace metals.

Crude title product (10.33 kg) was transferred to the dissolution vessel, and the mixture heated to near reflux (approximately 82°C). This temperature was maintained with agitation until all solid material was dissolved.

The hot isopropanol solution was transferred to a crystallizer through a cartridge filter. Hot isopropanol (78 to 82°C, about 10 L) was used to wash the dissolution vessel and the filter. The filter temperature was maintained just below 80°C. The volume of isopropanol wash was selected to ensure a thorough wash of the filter. The filtrate was concentrated to about 200 L at atmospheric pressure (near reflux, 80 to 82°C) prior to the following water addition to prevent yield loss, during which any solids precipitated after the filtration dissolved.

With moderate agitation, hot deionized water (80 to 85°C, 182 L) was slowly transferred to the crystallizer via an in-line filter. Crystallization started towards the end of this addition, and proceeded steadily. The crystal slurry was cooled to 0 to 5°C over 2 to 3 hours. This temperature was maintained for 1 additional hour. The crystal slurry was transferred to a centrifuge for filtration. Isopropanol (10 L) and deionized water (10 L) were

mixed, and transferred to the crystallizer via the filter. The mixture was transferred to the centrifuge as a wash of the crystalline product. The title product was dried at 50 to 60°C under vacuum in a dryer until constant weight was achieved.

EXAMPLE 7

Preparation of (11β,17β)-11-(Acetyloxy)-17-(ethylthio)-9-fluoro-17-(methylthio)androsta-1,4-dien-3-one

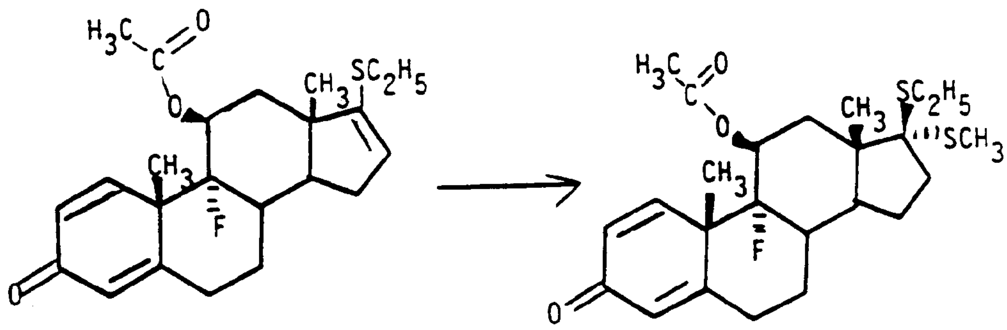

A solution of trichloroacetic acid (10.05 g, 61.5 mmol, 1.5 equiv.) and trichloroacetic anhydride (0.75 ml, 4.1 mmol, 0.1 equiv.) in 40 ml methylene chloride was heated at reflux under nitrogen for 0.5 hours and then cooled to -20°C.

A solution of (11β)-11-(acetyloxy)-9-fluoro-17-(ethylthio)-androsta-1,4,16-trien-3-one (16.594 g, 41.0 mmol, 1.0 equiv.) in methylene chloride (269 ml) was concentrated atmospherically to 225 ml to remove residual water. The solution was cooled to -10°C under nitrogen and methanethiol (11.4 ml, approximately 227 mmol, approximately 5.5 equiv.) (condensed in a graduated cylinder with a dry ice/isopropanol bath) was added. The solution was then cooled to -20°C and the trichloroacetic acid solution prepared above was added all at once. The reaction was maintained at -20°C under nitrogen and followed by HPLC to completion.

The reaction was quenched after 1.5 hours with 1.0N NaOH (256 ml, 256 mmol, 6.2 equiv.) and allowed to stir for 15 minutes. The mixture was transferred to a separatory funnel and the layers were separated. The aqueous layer was back-extracted with methylene chloride (40 ml). The organic layers were combined and the extraction sequence was repeated.

The organic layers were combined with diethylamine (0.9 ml, 8.7 mmol, 0.21 equiv.) added to decompose trichloroacetyl methylthioester formed by the reaction of trichloroacetic anhydride and methanethiol, and isopropyl alcohol (IPA) (128 ml) and concentrated atmospherically to 82°C. After diluting to approximately 150 ml with IPA, the solution was brought to reflux and distilled water (352 ml) at approximately 80°C was added dropwise to effect crystallization. The resulting slurry was cooled to approximately 5°C and held for 0.5 hours. The product was filtered, washed with 100 ml of 3:1 $H_2O$/IPA at approximately 5°C, and dried overnight at approximately 50°C to provide 18.378 g (99.0 M% uncorrected) of the title compound.

EXAMPLE 8

Preparation of (11β,17β)-17-(Ethylthio)-9-fluoro-11-hydroxy-17-(methylthio)androsta-1,4-dien-3-one

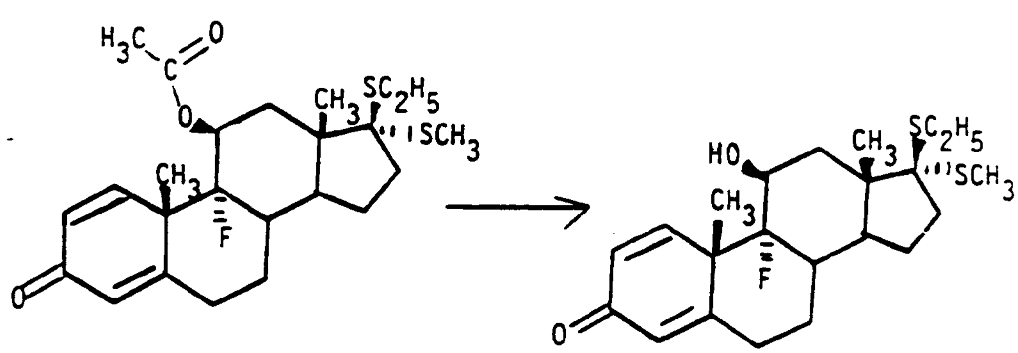

A slurry of (11β,17β)-11-(acetyloxy)-17-(ethylthio)-9-fluoro-17-(methylthio)androsta-1,4-dien-3-one (10.878 g, 24.0 mmol, 1.0 equiv.) prepared as in Example 7 above in 175 ml methanol was heated to reflux under nitrogen to effect dissolution. While maintaining reflux, 5.0 N NaOH (9.57 ml, 47.85 mmol, 2.0 equiv.) was added. After 42 minutes at reflux, the reaction was judged complete by HPLC, cooled several degrees and quenched by dropwise addition of glacial acetic acid (1.63 ml, 28.5 mmol, 1.2 equiv.).

Methanol was concentrated atmospherically to a minimum volume (approximately 70 ml) where water was added dropwise until the batch temperature reached 90°C. The resulting slurry was diluted to approximately 100 ml and cooled to approximately 5°C. After holding at approximately 5°C for 20 minutes, the product was filtered, washed with 50 ml $H_2O$ and dried overnight at approximately 50°C to provide 9.833 g (90.4 M% uncorrected) of the title compound.

The crude title compound was recrystallized from isopropyl alcohol (IPA) (325 ml) at reflux by slow addition of $H_2O$ (100 ml) at approximately 80°C and subsequent cooling to approximately 5°C. The product was filtered, washed with 50 ml of 3:1 IPA/$H_2O$ at 5°C, and dried overnight under vacuum at approximately 50°C to provide 8.87 g (90.2 W% uncorrected) of the title compound.

## EXAMPLE 9

Compounds of the formula VI as described in the following Table 3 are prepared by employing a procedure analogous to that of Example 5 above, where the thiol or starting material is substituted as indicated.

TABLE 3

| | Starting Material | Thiol | Formula VI Compound |
|---|---|---|---|
| | 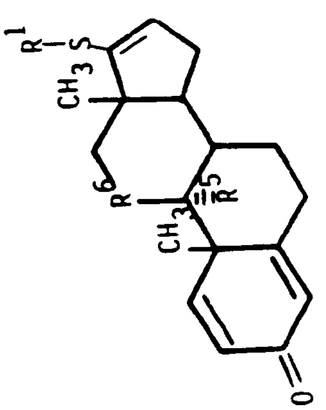 | $R^2$-SH | 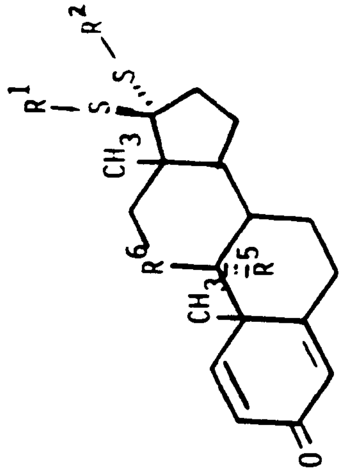 |

| | $R^1$ | $R^2$ | $R^5$ | $R^6$ |
|---|---|---|---|---|
| (a) | $C_2H_5$ | $C_6H_5$ | F | β-$CH_3COO$-CH |
| (b) | $C_6H_5$ | $C_2H_5$ | F | β-$CH_3COO$-CH |
| (c) | $C_2H_5$ | n-$C_4H_9$ | F | β-$CH_3COO$-CH |
| (d) | $CH_3$ | $C_2H_5$ | H | β-OH |
| (e) | $C_2H_5$ | $CH_3$ | H | β-OH |

EXAMPLE 10

Additional compounds of the formula VI are prepared by converting the (β- acetyloxy)methine group of the compounds prepared in (a), (b) and (c) of Example 9 above to a (β-hydroxy)methine group by a procedure analogous to that employed in Example 6 above.

EXAMPLE 11

Preparation of (11β,17α)-11-(Acetyloxy)-17-(ethylthio)-9-fluoro-17-(methylthio)androsta-1,4-dien-3-one with $BF_3Et_2O/CF_3COOH$ catalyst

(11β)-11-(Acetyloxy)-9-fluoro-17-(methylthio)androsta-1,4,16-trien-3-one (1.00 g) was dissolved in dry methylene chloride (15 mL) and brought to -40°C. Ethanethiol (0.21 g), boron trifluoride ethyl etherate (0.18 g), and trifluoroacetic acid (0.58 g) were added and the reaction allowed to proceed for five minutes. The reaction mixture, quenched by the addition of triethylamine (1 mL), contained the title product in roughly 96% yield.

EXAMPLE 12

Preparation of (11β,17α)-17-(Ethylthio)-9-fluoro-11-hydroxy-17-(methylthio)androsta-1,4-dien-3-one

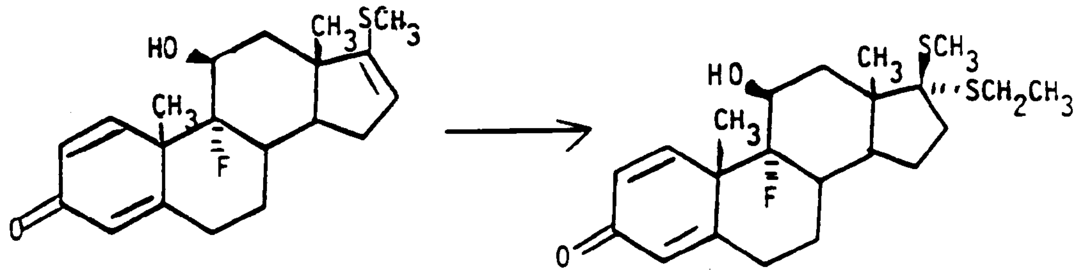

(11β)-11-(Hydroxy)-9-fluoro-17-(methylthio)androsta-1,4,16-trien-3-one (0.27 g, 0.77 mmol) was heated in a large test tube to 80°C. Dry methylene chloride (10 ml) was added along with trifluoroacetic anhydride (approximately 50 μl). The suspension was stirred for 2 minutes. Trifluoroacetic acid (1.00 ml) and trifluoroacetic anhydride (approximately 200 μl) were mixed thoroughly and allowed to stand for several minutes (capped). A portion of this solution (approximately 160 μl) was added to the suspension, causing dissolution of the starting material and formation of a pink color.

The solution was brought to -43°C for several minutes and ethanethiol (approximately 200 μl) was added. The solution was allowed to stir for 0.5 hour at -43 to -40°C. The reaction was quenched by the addition of sodium hydroxide (3 pellets amounting to 0.360 g) dissolved in water (approximately 0.5 ml) with methanol (approximately 1.5 ml) added. The solution was stirred vigorously for 5 minutes, and then allowed to come to room temperature. 45 Minutes after quenching, the layers were separated and the methylene chloride layer re-extracted with water, and then stripped to dryness (0.32 g).

The above material was re-crystallized from hot ethanol (approximately 10 ml) to which water (approximately 4 ml) was added after filtering (a small amount (approximately 30 mg) DARCO was employed). (0.31 g, dry).

255 mg of the crude title product obtained were dissolved in hot isopropanol (4.6 ml). Water (4.6 ml) was gradually added, and the material allowed to slowly crystallize. Obtained were 239 mg of the title product (approximately 94% on recrystallization). (m.p. 225-226°C).

## Claims

1. A process for the preparation of a mixed 3-keto,17-thioketalandrostene of the formula VI:

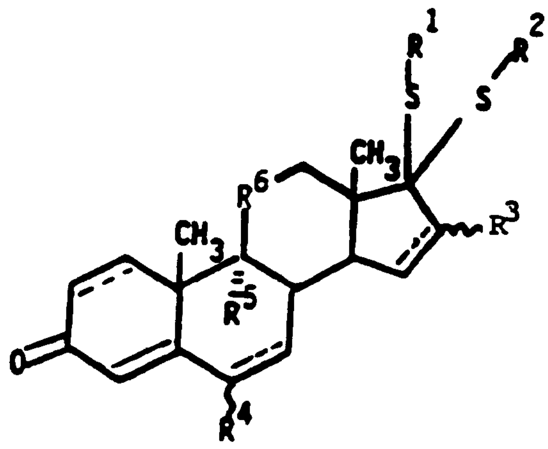

(VI)

where

$R^1$ and $R^2$ are different, and are alkyl, cycloalkyl or aryl;

$R^3$ is hydrogen, alkoxy, aryloxy, alkylthio, arylthio, alkylcarbonyloxy or halogen, or, when the 15,16-positions are saturated, hydroxy;

$R^4$ is hydrogen, alkyl, hydroxy, alkylcarbonyloxy or halogen;

$R^5$ is hydrogen or halogen; and

$R^6$ is carbonyl or (β-hydroxy)methine,

comprising the steps of:

(a) preparing a 3-keto,17-thioenoletherandrostene of the formula IV:

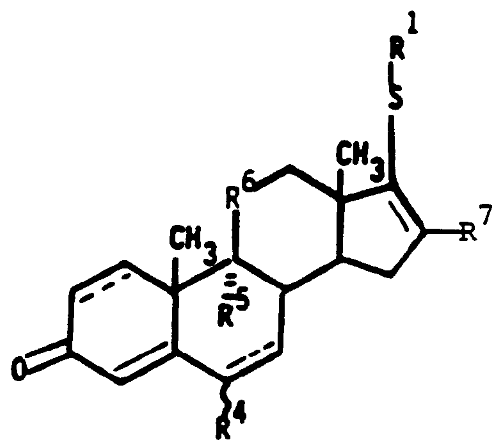

(IV)

where

$R^7$ is hydrogen, alkoxy, aryloxy, alkylthio, arylthio, alkylcarbonyloxy or halogen;

$R^1$, $R^4$ and $R^5$ are as defined above for said formula VI; and

$R^6$ is carbonyl, (β-hydroxy)methine or protected (β-hydroxy)methine,

by a process comprising the step of contacting a 3-keto,17-thioketalandrostene of the formula V:

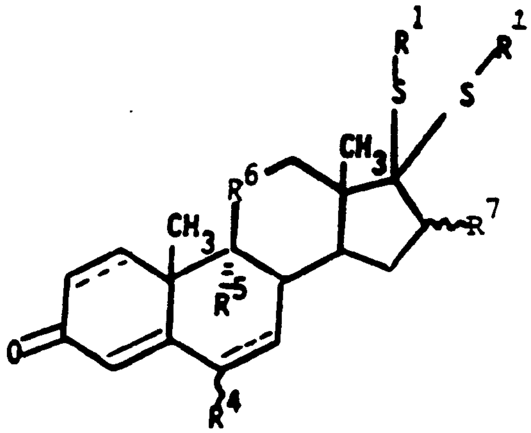

(V)

where $R^1$, $R^7$, $R^4$, $R^5$ and $R^6$ are as defined above for said formula IV,and where both $R^1$ groups are the same,
with a protic acid catalyst; and
(b) converting the 3-keto,17-thioenoletherandrostene of said formula IV prepared in step (a) to the mixed 3-keto,17-thioketalandrostene of said formula VI.

**2.** The process of claim 1, wherein the 3-keto,17-thioketalandrostene of said formula V, employed as the starting material in step (a), is prepared from the correnponding 3,17-diketoandrostene by a process comprising the step of contacting said 3,17-diketoandrostene with a thiol of the formula $R^1$-SH, where $R^1$ is as defined above for said formula IV,in the presence of a protic acid catalyst which is a complex of a Lewis acid and a protic source, wherein the temperature of the reaction medium is maintained below about 25°C during the reaction, and, optionally, (i) an excess of said acid catalyst is employed and/or (ii) an excess of said thiol is employed.

**3.** The process of claim 1 or 2, wherein said formula VI compound is a
$\Delta^{1,4}$-androstadiene where,
$R^1$ is β-methyl;
$R^2$ is α-ethyl;
$R^3$ and $R^4$ are hydrogen;
$R^5$ is fluoro; and,
$R^6$ is (β-hydroxy)methine.

**4.** A process for the preparation of a thioketal from a thioenolether, comprising the step of contacting said thioenol ether with a thiol in the presence of a protic acid catalyst.

**5.** The process of claim 4, wherein a mixed thioketal is prepared.

**6.** The process of claim 5, wherein a mixed 3-keto,17-thioketalandrostene is prepared from the corresponding 3-keto,17-thioenoletherandrostene.

**7.** The process of claim 6, wherein a mixed 3-keto,17-thioketalandrostene of the formula VI is prepared:

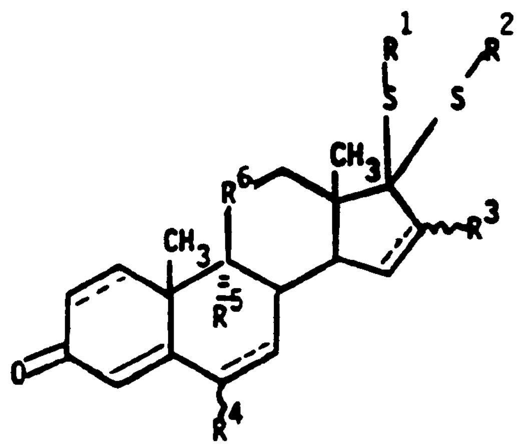

(VI)

where

$R^1$ and $R^2$ are different, and are alkyl, cycloalkyl or aryl;

$R^3$ is hydrogen, alkoxy, aryloxy, alkylthio, arylthio, alkylcarbonyloxy or halogen or, when the 15, 16-positions are saturated, hydroxy;

$R^4$ is hydrogen, alkyl, hydroxy, alkylcarbonyloxy or halogen;

$R^5$ is hydrogen or halogen; and

$R^6$ is carbonyl, (β-hydroxy)methine or protected (β-hydroxy)methine,

by a process comprising the step of contacting a 3-keto,17-thioenoletherandrostene of the formula IV:

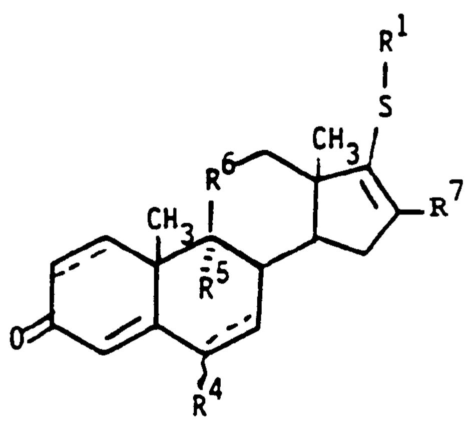

(IV)

where

$R^7$ is hydrogen, alkoxy, aryloxy, alkylthio, arylthio, alkylcarbonyloxy or halogen; and

$R^1$, $R^4$, $R^5$ and $R^6$ are as defined above for said formula VI, with a thiol of the formula VII:

$R^2$-SH (VII)

where $R^2$ is as defined above for said formula VI, in the presence of a protic acid catalyst.

**8.** The process of claim 7, wherein a mixed 3-keto,17-thioketalandrostene of the formula VIa is prepared:

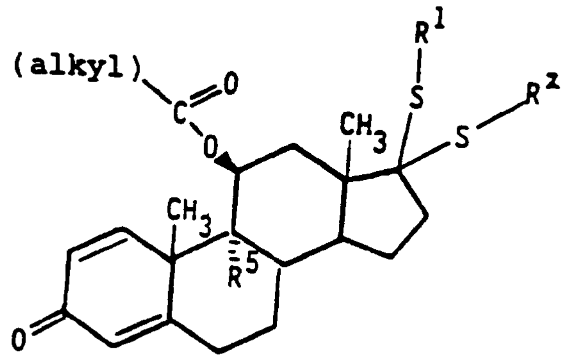

(VIa)

where $R^1$ an $R^2$ are different and are alkyl; and

$R^5$ is halogen,

by a process comprising the step of contacting a 3-keto,17-thioenoletherandrostene of the formula IVa:

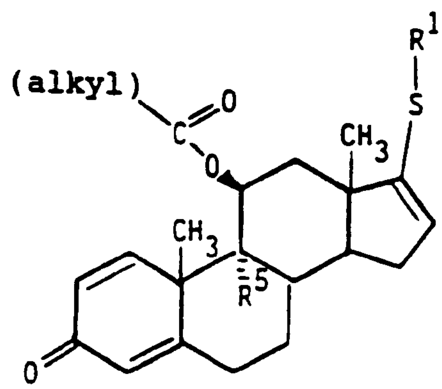

(IVa)

where $R^1$ and $R^5$ are as defined above for said formula VIa with a thiol of the formula VIIa:

$R^2$-SH (VIIa)

where $R^2$ is as defined above for said formula VIa, in the presence of a protic acid catalyst.

**9.** The process of claim 8, wherein said 3-keto,17-thioenoletherandrostene starting material of the formula IV is prepared from the corresponding 3-keto,17-thioketalandrostene in which the 17-position is disubstituted with the same thioether group, by a process comprising the step of contacting said 3-keto,17-thioketalandrostene in which the 17-position is disubstituted with the same thioether group with a protic acid catalyst.

**10.** The process of claim 9, wherein said 3-keto,17-thioketalandrostene in which the 17-position is disubstituted with the same thioether group is prepared from the corresponding 3,17-diketoandrostene, by a process comprising the step of contacting said 3,17-diketoandrostene with a thiol in the presence of a protic acid catalyst which is a complex of a Lewis acid and a protic source, wherein the temperature of the reaction medium is maintained below about 25°C during the reaction, and, optionally, (i) said acid catalyst is employed in excess, and/or (ii) said thiol is employed in excess.

**11.** The process of any one of claims 7-10, wherein said protic acid catalyst is a trihaloacetic acid, an organic sulfonic acid of the formula R-$SO_3H$ where R is alkyl or aryl, phosphoric acid, or a Lewis acid/protic source complex.

**12.** The process of claim 11, wherein said protic acid catalyst is a trihaloacetic acid.

**13.** The process of claim 12,wherein said protic acid catalyst is trichloroacetic acid.

**14.** The process of claim 12,wherein said trihaloacetic acid is mixed, prior to or during said process, with the corresponding acid anhydride thereof.

**15.** The process of claim 11, wherein water is not the sole protic source of said protic acid catalyst.

**16.** The process of any one of claims 7-15, wherein said protic acid catalyst has a $pK_a$ less than or equal to that $pK_a$ at which said 3-keto,17-thioenoletherandrostene starting material will first accept and become activated by a proton, and greater than or equal to about -10.

**17.** The process of claim 16, wherein said protic acid catalyst has a pKa of from about 2.5 to about -4.0.

**18.** The process of claim 17, wherein a mixed 3-keto,17-thioketalandrostene of the formula VI having the following designated stereoisomeric configuration:

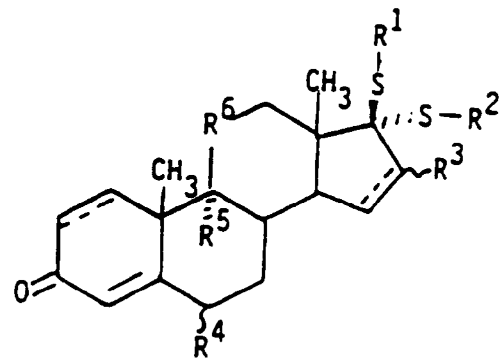

is prepared preferentially, relative to its 17-position stereoisomer.

**19.** The process of claim 18, wherein said mixed 3-keto,17-thioketalandrostene having said designated stereoisomeric configuration is prepared substantially free of its 17-position isomer.

**20.** The process of any one of claims 7-19, wherein a mixed 3-keto,17-thioketal-$\Delta^{1,4}$-androstadiene of the formula VI is prepared in which:

$R^1$ is β-methyl;
$R^2$ is α-ethyl;
$R^3$ and $R^4$ are hydrogen;
$R^5$ is fluoro; and
$R^6$ is protected (β-hydroxy)methine, wherein said mixed androstadiene is subsequently deprotected at the 11-position to yield the corresponding (β-hydroxy)methine compound.

**21.** A selective thioketalization process for the preparation of a thioketal from a dione where one, but not the other, keto group of the dione is α,β-unsaturated, and where selective thioketalization occurs at the keto group of the dione which is not α,β-unsaturated, comprising the step of contacting said dione with a thiol, in the presence of a protic acid catalyst which is a complex of a Lewis acid and a protic source, wherein the temperature of the reaction medium is maintained below about 25°C during the reaction.

**22.** The process of claim 21,wherein (i) said acid catalyst is employed in excess and/or (ii) said thiol is employed in excess.

**23.** The process of claim 21, wherein a 3-keto,17-thioketalandrostene is prepared from the corresponding 3,17-diketoandrostene.

**24.** The process of claim 23, wherein a 3-keto,17-thioketalandrostene of the formula I is prepared:

(I)

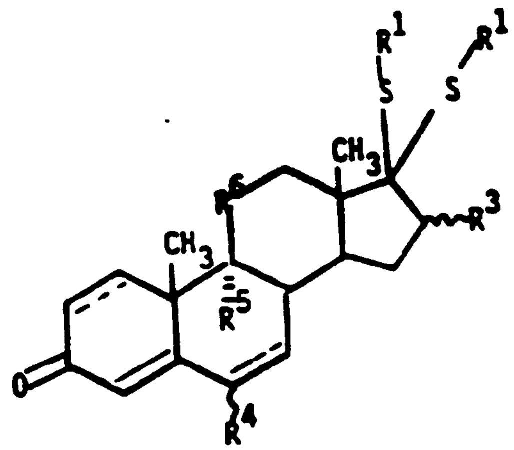

where

$R^1$ is alkyl, cycloalkyl or aryl, and both $R^1$ groups are the same;

$R^3$ is hydrogen, hydroxy, alkoxy, aryloxy, alkylthio, arylthio, alkylcarbonyloxy or halogen;

$R^4$ is hydrogen, alkyl, hydroxy, alkylcarbonyloxy or halogen;

$R^5$ is hydrogen or halogen; and

$R^6$ is carbonyl, (β-hydroxy)methine or protected (β-hydroxy)methine,

and wherein said process comprises the step of contacting a 3,17-diketoandrostene of the formula II:

(II)

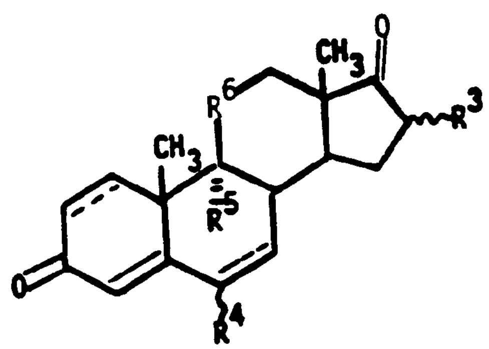

where $R^3$, $R^4$, $R^5$ and $R^6$ are as defined above for said formula I, with a thiol of the formula III:

$R^1$-SH (III)

where $R^1$ is as defined above for said formula I.

**25.** The process of claim 24, wherein a 3-keto,17-thioketalandrostene of the formula Ia is prepared:

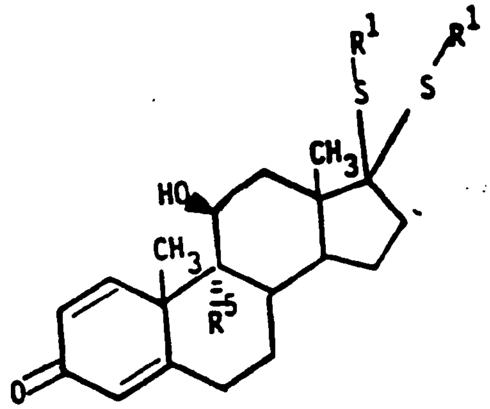

(Ia)

where

$R^1$ is alkyl, and both $R^1$ groups are the same; and

$R^5$ is halogen,

and wherein said process comprises the step of contacting a 3,17-diketoandrostene of the formula IIa:

(IIa)

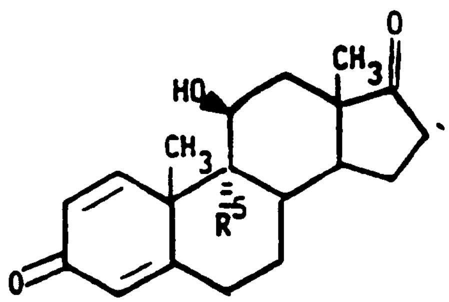

where $R^5$ is as defined above for said formula Ia, with a thiol of the formula IIIa:

$R^1$-SH (IIIa)

where $R^1$ is as defined above for said formula Ia.

**26.** The process of claim 24 or 25, wherein (i) said acid catalyst is employed in excess and/or (ii) said thiol is employed in excess.

**27.** The process of claim 26, wherein both said conditions (i) and (ii) are employed.

**28.** The process of claim 26 or 27, wherein the molar ratio of said acid catalyst to said 3,17-diketoandrostene is from about 2:1 to about 10:1.

**29.** The process of claim 26, 27 or 28, wherein the molar ratio of said thiol to said 3,17-diketoandrostene is from about 3:1 to about 10:1.

**30.** The process of any one of claims 20-29, wherein said Lewis acid is boron trifluoride or an etherate thereof, or is $SnCl_4$, $TiCl_4$, $BBr_3$, or $BCl_3$, and said protic source is an organic alcohol or an organic protic acid.

**31.** The process of claim 30, wherein said Lewis acid is boron trifluoride or an etherate thereof.

**32.** The process of claim 30 or 31, wherein said protic source is an alkyl carboxylic acid.

**33.** The process of claim 31, wherein said Lewis acid is boron trifluoride ethyl etherate and said protic source

is acetic acid.

**34.** The process of any one of claims 21-33, wherein the temperature of said reaction medium is maintained below-about 15°C.

**35.** The process of claim 34, wherein the temperature of said reaction medium is maintained below about 10°C.

**36.** The process of any one of claims 21-35, wherein said temperature is maintained at least in part by pre-cooling one or more of the reaction components.

**37.** A process for the preparation of a mixed 3-keto,17-thioketalandrostene of the formula VI:

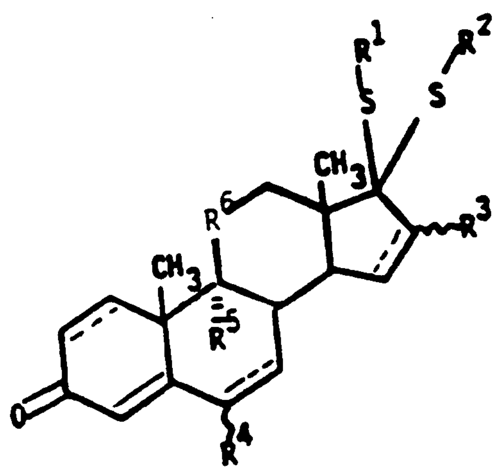

(VI)

where

$R^1$ and $R^2$ are different, and are alkyl, cycloalkyl or aryl;

$R^3$ is hydrogen, alkoxy, aryloxy, alkylthio, arylthio, alkylcarbonyloxy or halogen, or, when the 15,16-positions are saturated, hydroxy;

$R^4$ is hydrogen, alkyl, hydroxy, alkylcarbonyloxy or halogen;

$R^5$ is hydrogen or halogen; and

$R^6$ is carbonyl or (β-hydroxy)methine,

comprising the steps of

(a) carrying out the process of claim 24 or any claim dependent thereon; and

(b) converting the 3-keto,17-thioketalandrostene of said formula I prepared in step (a) to the mixed 3-keto,17-thioketalandrostene of said formula VI.

**38.** The process of claim 37, wherein said formula VI compound is a $\Delta^{1,4}$-androstadiene wherein

$R^1$ is β-methyl;

$R^2$ is α-ethyl;

$R^3$ and $R^4$ are hydrogen;

$R^5$ is fluoro; and

$R^6$ is (β-hydroxy)methine.

**39.** A process for the preparation of a 3-keto,17-thioenoletherandrostene from the corresponding 3-keto,17-thioketalandrostene, comprising the step of contacting said 3-keto,17-thioketalandrostene with a protic acid catalyst.

**40.** The process of claim 39, wherein a 3-keto,17-thioenoletherandrostene of the formula IV is prepared:

(IV)

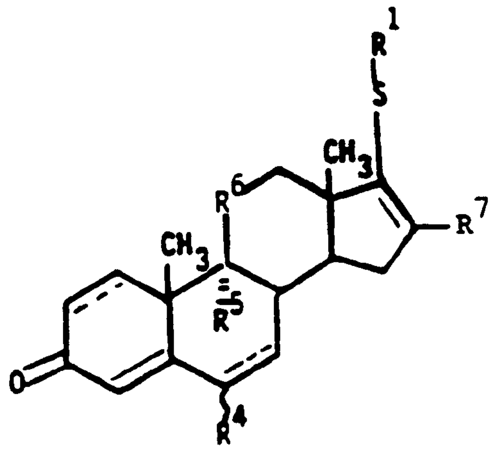

where

$R^1$ is alkyl, cycloalkyl or aryl;

$R^7$ is hydrogen, alkoxy, aryloxy, alkylthio, arylthio, alkylcarbonyloxy or halogen;

$R^4$ is hydrogen, alkyl, hydroxy, alkylcarbonyloxy or halogen;

$R^5$ is hydrogen or halogen; and

$R^6$ is carbonyl, (β-hydroxy)methine or protected (β-hydroxy)methine,

and wherein said process comprises the step of contacting a 3-keto,17-thioketalandrostene of the formula V:

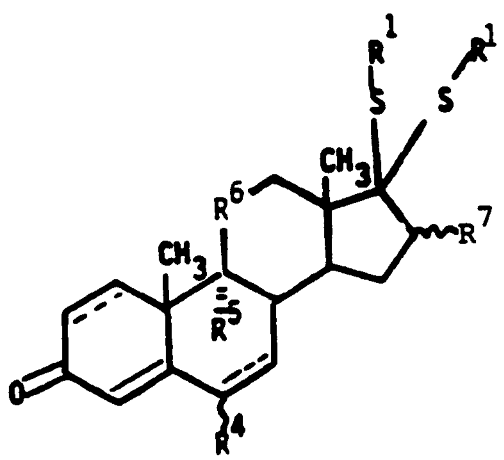

(V)

where

$R^1$, $R^7$, $R^4$, $R^5$ and $R^6$ are as defined above for said formula IV and both $R^1$ groups are the same, with a protic acid catalyst.

**41.** The process of claim 40, wherein a 3-keto,17-thioenoletherandrostene of the formula IVa is prepared:

(IVa)

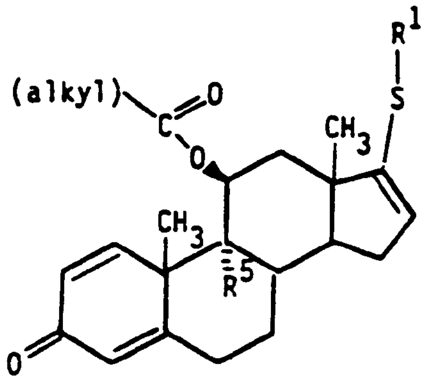

where

R[1] is alkyl; and

R[5] is halogen,

and wherein said process comprises the step of contacting a 3-keto,17-thioketalandrostene of the formula Va:

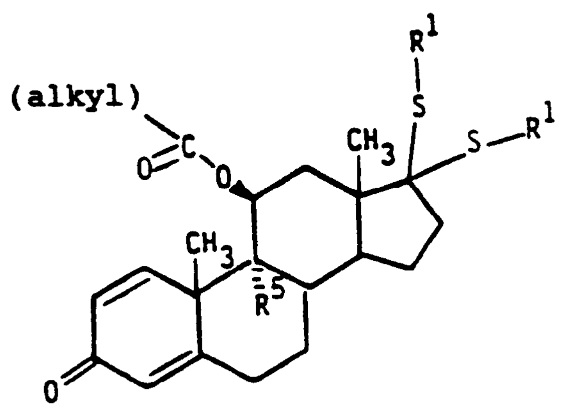

(Va)

where R[1] and R[5] are as defined above for said formula IVa, and both R[1] groups are the same, with a protic acid catalyst.

42. The process of claim 40, wherein said 3-keto,17-thioketalandrostene starting material of the formula V is prepared from the corresponding 3,17-diketoandrostene by a process according to claim 24 or any claim dependent thereon.

43. The process of any one of claims 39-42,wherein said acid catalyst has a pKa less than or equal to about 2.5.

44. The process of any one of claims 39-43, wherein said acid catalyst is a Lewis acid/protic source complex.

45. The process of claim 44, wherein said Lewis acid is boron trifluoride or an etherate thereof.

46. The process of claim 44 or 45, wherein said protic source is an alkyl carboxylic acid.

47. The process of claim 46, wherein said Lewis acid is boron trifluoride ethyl etherate and said alkyl carboxylic acid is acetic acid.

48. The process of any one of claims 39-47, wherein the rate of reaction is controlled so that the function of product concentration versus time is closer to linear than in the absence of the control.

49. The process of claim 48, wherein control of said rate of reaction is achieved by control of the rate of addition

of said acid catalyst.

**50.** The process of any .one of claims 39-49, wherein at least part of the thiol $R^1$-SH, released from said 3-keto,17-thioketalandrostene starting material during said process, is removed during the reaction.

**51.** A process for the preparation of a thioenol ether from a thioketal, comprising the step of contacting said thioketal with a protic acid catalyst, wherein said catalyst is formed, at least in part, by the combination of a protic acid precursor with a protic source.

**52.** The process of claim 51, wherein the use of said precursor, at least in part, renders ineffective as a hydrolysis agent water present in one or more of the reaction components.

**53.** The process of claim 51 or 52, wherein said protic source is other than a thiol having the structure of a thiol released from said thioketal starting material during the reaction.

**54.** A process for the preparation of a thioenol ether from a thioketal, comprising the step of contacting said thioketal with a protic acid catalyst, where the reaction rate is controlled so that the function of product concentration versus time is closer to linear than in the absence of the control and/or where the thiol released from said thioketal starting material is removed during said reaction.

**55.** A process for the preparation of a thioenol ether from a thioketal, comprising the step of contacting said thioketal with a protic acid catalyst, where said thioketal starting material is prepared in a separate step prior to the preparation of said thioenol ether.

European Patent Office

# EUROPEAN SEARCH REPORT

Application Number

EP 92306711.0

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| A | EP - A - 0 073 026 (SQUIBB) * Pages 1-10 * | 1-55 | C 07 J 31/00 |
| A | DE - A - 3 324 403 (SQUIBB) * Pages 4-9 * | 1-55 | |
| D,A | US - A - 4 361 559 (R.K. VARMA) * Totality * | 1-55 | |

TECHNICAL FIELDS SEARCHED (Int. Cl.5)

C 07 J 31/00

The present search report has been drawn up for all claims

| Place of search | Date of completion of the search | Examiner |
|---|---|---|
| VIENNA | 08-10-1992 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)